# EUROPÄISCHE PATENTSCHRIFT

(11) **EP 1 296 930 B1**
(45) Veröffentlichungstag und Bekanntmachung des Hinweises auf die Patenterteilung: **19.08.2009**
(21) Anmeldenummer: 01962766.0
(22) Anmeldetag: 22.06.2001
(51) Int. Cl.: C07C 209/60, C07C 209/64

(54) **VERFAHREN ZUR HERSTELLUNG VON ALKYLAMINEN**
METHOD FOR PRODUCING ALKYLAMINES
PROCEDE DE PRODUCTION D'ALKYLAMINES

(30) Priorität: 28.06.2000 DE 10030619; 24.08.2000 DE 10041676
(43) Veröffentlichungstag der Anmeldung: 02.04.2003
(73) Patentinhaber: BASF SE, 67056 Ludwigshafen (DE)
(72) Erfinder: FUNKE, Frank, 68159 Mannheim (DE); STEINBRENNER, Ulrich, 67435 Neustadt (DE); FRAUENKRON, Matthias, 67251 Freinsheim (DE); BÖHLING, Ralf, 64347 Griesheim (DE); MELDER, Johann-Peter, 67459 Böhl-Iggelheim (DE); HEIDEMANN, Thomas, 68519 Viernheim (DE)
(86) Internationale Anmeldenummer: PCT/EP2001/007124
(87) Internationale Veröffentlichungsnummer: WO 2002/000597

(56) Entgegenhaltungen:
- EP-A- 0 039 918
- EP-A- 0 054 746
- DE-C- 510 439
- DE-C- 697 372
- FR-A- 1 484 782

## Beschreibung

Die vorliegende Erfindung betrifft ein Verfahren zur Herstellung von Alkylaminen.

Alkylamine dienen als Ausgangsstoffe für die Herstellung von Tensiden, Textil- und Flotationshilfsmitteln, Bakteriziden, Korrosions- und Schauminhibitoren, Additiven für Pharmazeutika sowie als Antioxidantien für Fette und Öle.

Alkylamine können durch die Hydrierung von entsprechenden Nitrilen oder Nitroverbindungen, durch die reduktive Aminierung von entsprechenden Aldehyden und Ketonen und durch die Aminierung von entsprechenden Alkoholen hergestellt werden.

Die niederen Alkylamine (C₁- bis C₁₀-Alkylamine), wie die Ethylamine, Butylamine und Isopropylamine, werden technisch insbesondere durch die Aminierung des korrespondierenden Alkohols oder der korrespondierenden Carbonylverbindung an Metallkatalysatoren, die z. B. geträgert sind, unter hydrierenden Bedingungen hergestellt (siehe z. B.: Ullmann's Encyclopedia of Industrial Chemistry, Vol. A 2, 5th Ed., Seite 4).

In FR-A-1 484 782 ist eine Umalkylierung beschrieben, bei der aus einer Mischung von primären, sekundären und/oder tertiären Aminen durch Kondensation, d.h. unter Abspaltung von Ammoniak, der Anteil von sekundären und tertiären Aminen in der Mischung erhöht wird.

Alternativ können Alkylamine auch an sauren Phosphatkatalysatoren aus entsprechenden Alkoholen hergestellt werden (siehe z. B. US-A-4,582,904 (Air Products)).

Eine weitere Alternative zur Herstellung von Alkylaminen besteht in der Addition von NH₃ oder Aminen an Olefine in Gegenwart von sauren Katalysatoren, wie z. B. Zeolithen (siehe z. B. EP-A-132 736) oder Aluminosilikaten (siehe z. B. EP-A1-0039918, in der die katalytische Herstellung von Aminen in der Gasphase durch Umsetzung von bestimmten Olefinen mit Ammoniak, primären oder sekundären Aminen in Gegenwart von Aluminosilikaten offenbart wird), in Gegenwart von basischen Katalysatoren, wie z.B. Metallamiden, insbesondere Alkali- und Erdalkalimetallamiden, (siehe z. B. B.W. Howk et. al., J. Am. Chem. Soc. 76, Seite 1899ff (1954) ; R. Stroh et al., Angew. Chem. 69, 124ff (1957)oder EP-A1-0054746, in der die Hydroaminierung von Monoolefinen mit Ammoniak in Gegenwart von Alkalimetallamid-Katalysatoren beschrieben wird), Amiden der IV. Nebengruppe (siehe z. B. D. Steinborn et al. (Z. Chem. 29 (1989), Seite 333ff) oder Alkalialkoholaten, oder in Gegenwart von Übergangsmetallkomplexverbindungen (siehe z. B. US-A-3,758,586).

Diese Alternative wird technisch aber bisher kaum genutzt.

Die oben angeführten Verfahren zur Herstellung von Alkylaminen weisen folgende Nachteile auf:

DerEinsatzvonAlkohlen (z. B. Ethanol), Aldehyden, KetonenundNitrilen als Ausgangsstoffe (Edukte) für die Herstellung von Alkylaminen ist bezogen auf deren Preise wesentlich unwirtschaftlicher als der Einsatz von entsprechenden Olefinen (z. B. Ethen).

Der Einsatz von Olefinen als Ausgangsstoff für die Alkylaminherstellung ist demnach wünschenswert, jedoch bisher mit folgenden Nachteilen behaftet (vgl. z. B.: M. Beller et al., Chem. Rev. 98, 675f (1998) 675; R. Taube, ,Reaction with Nitrogen Compounds' in B. Cornils und W. A. Hermann: Applied Homogeneous Catalysis with Organometallic Compounds', VCH Weinheim, 1996, Seiten 507 bis 520, und E. Haak et al., Chemie in unserer Zeit (1999), 297 bis 303, insbesondere die. Zusammenfasung auf S. 302):
aa) Die basisch heterogen katalysierte Addition von Aminen an Olefine an Metalloxiden gelingt nach Kakuno et al. (J. Catal. 85 (1984), Seite 509ff) mit primären und sekundären Alkylaminen und konjugierten Dienen wie Butadien oder Isopren; der allgemeine Einsatz von NH₃ oder Monoolefinen ist nicht beschrieben.
ab) Die schwach basisch katalysierte Addition von Aminen an Olefine mit Alkalialkoholat als Katalysator ist nach Beller et al. (Angew. Chem. 110 (1998), Seite 3571ff) erfolgreich im Falle von aromatisch konjugierten Aminen und Styrol als Olefinkomponente. Im Falle von NH₃ bzw. Monoolefin als Edukt sind die Katalysatoren inaktiv.
ac) Bei der NaNH₂- oder KNH₂-katalysierten Addition von NH₃ an Olefine, wie sie z. B. inB.W. Howk et al., J. Am. Chem. Soc. 76 (1954), 1899-1902 und R.D. Closson et al., US-A-2,750,417 beschrieben ist, sind die Raum-Zeit-Ausbeuten an gewünschten Alkylaminen selbst bei hohen Temperaturen und Olefindrucken aufgrund der geringen Aktivität und Löslichkeit des Metallamids sehr klein.
ad) G.P. Pez (US-A-4,336,162 und US-A-4,302,603) beschreibt einen Lösungsansatz für dieses Problem durch den Wechsel zu den Rb- und Cs-Amiden oder die Verwendung eines Eutektikums von NaNH₂ und KNH₂. Im ersten Fall verbietet sich die technische Realisierung wegen des extrem hohen Katalysatorpreises, im zweiten Fall sind die Raum-Zeit-Ausbeuten an gewünschten Alkylaminen immer noch zu klein.
ae) Alkalimetallmonoalkylamide oder Alkalimetalldialkylamide sind als starke Basen zwar für die Addition von Olefinen, wie z. B. Ethylen, an Amine mit ausreichenden Raum-Zeit-Ausbeuten einsetzbar, in Gegenwart von NH₃ findet jedoch sofortige Protolyse des entsprechenden Alkalimetallalkylamids zu MNH₂ (M = Alkalimetall) statt. Dieses besitzt wiederum die bereits oben ausgeführten Nachteile.

Die Alkalimetallalkylamid-katalysierte Addition von Aminen an Olefine in Abwesenheit von NH₃ besitzt aber wiederum den Nachteil, dass statt dem preiswerten NH₃ teurere Amine als Edukte eingesetzt werden müssen.
b) An sauren Katalysatoren, wie z.B. Zeolithen, gelingt die Addition - von NH₃ an Olefine, wobei Ammoniak in der Regel in hohem Überschuss bezogen auf das Olefin eingesetzt wird, nicht in jedem Fall mit so guten Selektivitäten und Ausbeuten'für ein bestimmtes Alkylamin wie z. B. im Fall von Isobuten (siehe z. B. DE-A-36 34 247).

So fanden z. B. M. Deeba et al. in Zeolites 10 (1990), Seite 794ff, und in Chem. Ind. 40 (1990), Seite 241ff, bei der Umsetzung von Ethylen und NH₃ an Zeolithen und Gardner et al. in EP-A-200 923 bei der Umsetzung von Ethylen und NH₃ in Gegenwart von NH₄I hauptsächlich Mono- neben geringen Mengen an Di- und sehr geringen Mengen an Triethylamin.

Ein Ausweg wäre die Hydroaminierung von Olefinen mit NH₃ im Unterschuss, doch hier erreicht man bezogen auf das Olefin in der Regel schlechte Selektivitäten und erzeugt eine rasche Desaktivierung des Katalysators.

Die Hydroaminierung von Olefinen mit sekundären Aminen in Gegenwart saurer Katalysatoren verläuft wiederum im allgemeinen in schlechteren Ausbeuten und mit schlechteren Selektivitäten als die entsprechende Hydroaminierung mit Ammoniak oder primären Aminen.
c) Die Übergangsmetallkomplex-katalysierte Hydroaminierung von Olefinen gelingt im allgemeinen nur mit sekundären Alkylaminen in guten Ausbeuten (z. B.: Brunet, Gazzetta Chimica Italiana, 127, 1997, Seiten 111 bis 118, Seite 112, linke Spalte) .Der vorliegenden Erfindung lag die Aufgabe zugrunde, unter Überwindung der Nachteile des Stands der Technik ein alternatives, wirtschaftliches und flexibles Verfahren zur Herstellung von Alkylaminen aufzufinden, das es ermöglicht, ein gewünschtes Alkylamin oder mehrere gewünschte Alkylamine mit hoher Raum-Zeit-Ausbeute und Selektivität herzustellen.

Demgemäß wurde ein Verfahren zur Herstellung von Alkylaminen gefunden, welches dadurch gekennzeichnet ist, dass man in einer ersten Verfahrensstufe ein Olefin
a) mit einem primären Amin und/oder einem sekundären Amin in Gegenwart eines Metallmonoalkylamids oder Metalldialkylamids als Katalysator oder
b) mit Ammoniak und/oder einem primären Amin in Gegenwart einer anorganischen Festkörpersäure als Katalysator oder
c) mit Ammoniak, einem primären Amin und/oder einem sekundären Amin in Gegenwart einer Übergangsmetallkomplexverbindung als Katalysator,
   unter hydroaminierenden Bedingungen umsetzt und anschließend das oder die erhaltene/n Hydroaminierungsprodukt/e in einer zweiten Verfahrensstufe unter umalkylierenden Bedingungen
d) in Gegenwart eines Umalkylierungskatalysators oder
e) in Gegenwart von Wasserstoff und eines umalkylierenden Hydrier- oder Dehydrierkatalysators
bei Temperaturen von 80 bis 400 °C umsetzt.

In einer bevorzugtenAusführungsform des Verfahrens werden in der Bilanz von den Einsatzstoffen Olefin und Ammoniak, primäres Amin und/oder sekundäres Amin nur Ammoniak und Olefin verbraucht, indem entsprechende Rückführströme an aus der ersten und/oder zweiten Verfahrensstufe erhaltenen Aminen in den Zulauf der ersten und/oder zweiten Verfahrensstufe eingestellt werden.

Im erfindungsgemäßen Verfahren einsetzbare Olefine oder Mischungen hiervon sind im allgemeinen Olefine der Formel 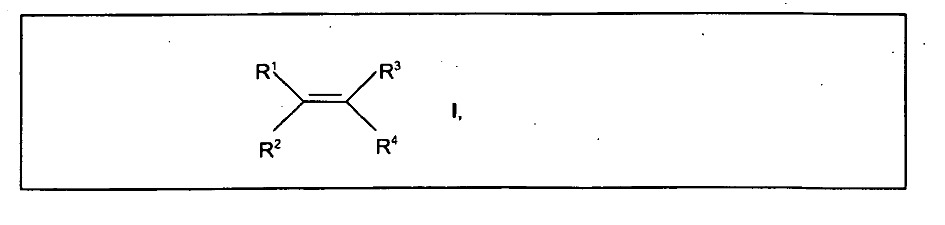 in der
R¹, R², R³, R⁴ Wasserstoff (H), C₁- bis C₂₀-Alkyl, C₂- bis C₂₀-Alkenyl, C₃- bis C₂₀-Cycloalkyl, C₅- bis C₈-Cycloalkenyl, C₆- bis C₂₀-Alkylcycloalkyl, C₆- bis C₂₀-Cycloalkyl-alkyl, Aryl, C₇- bis C₂₀-Alkylaryl und C₇- bis C₂₀-Aralkyl bedeuten und
R¹ und R³ zusätzlich gemeinsam eine C₂- bis C₁₂-Alkylenkette bedeuten kann.

Beispiele für solche Olefine sind Ethen, Propen, 1-Buten, 2-Buten, Isobuten, 1-Penten, 2-Penten, 1-Hexen, 2-Hexen, 3-Hexen, 1-Octen, Isoocten, 1-Decen, Styrol, Stilben, Cyclopenten, Cyclohexen, Allen, 1,3-Butadien, Isopren und 4-Vinyl-1-cyclohexen.

Im erfindungsgemäßen Verfahren einsetzbare primäre und sekundäre Amine oder Mischungen hiervon sind im allgemeinen Amine der Formel 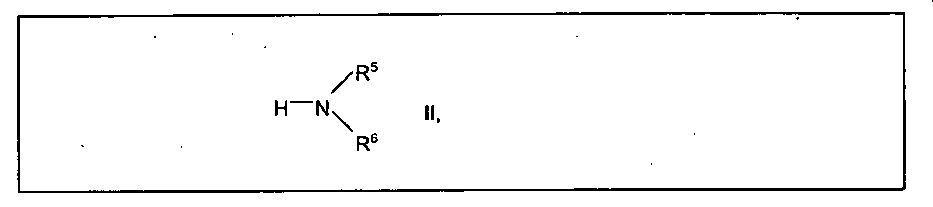 in der
R⁵ Wasserstoff (H), C₁- bis C₂₀-Alkyl, C₂- bis C₂₀-Alkenyl, C₃- bis C₂₀-Cycloalkyl, Alkoxyalkyl, Aminoalkyl, Monoalkylaminoalkyl, Dialkylaminoalkyl, Aralkyl
und R⁶ C₁- bis C₂₁-Alkyl, C₂- bis C₂₀-Alkenyl, C₃- bis C₂₀-Cycloalkyl, Alkoxyalkyl, Aminoalkyl, Monoalkylaminoalkyl, Dialkylaminoalkyl, Aralkyl bedeuten und
R⁵ und R⁶ gemeinsam eine gesättigte oder ungesättigte C₃- bis C₉-Alkylenkette, die durch ein O-, S- oder N-Heteroatom unterbrochen sein kann, insbesondere gemeinsam eine -(CH₂)ⱼ-X-(CH₂)ₖ- Gruppe, mit j und k = 1 bis 4 und X = CH₂, CHR⁷, Sauerstoff (O), Schwefel (S) oder NR⁷, mit R⁷ = H oder C₁- bis C₄-Alkyl, bedeuten kann.

Beispiele für solche primären Amine sind Methylamin, Ethylamin, n-Propylamin, iso-Propylamin, n-Butylamin, iso-Butylamin, tert.-Butylamin, n-Pentylamin, Cyclopentylamin, iso-Pentylamin, n-Hexylamin, Cyclohexylamin, n-Octylamin, n-Decylamin, 1-Phenylethylamin, 2-Phenylethylamin, Allylamin, 2-Dimethylaminoethylamin und 2-Methoxyethylamin.

Beispiele für sekundäre Amine gemäß Formel II sind Dimethylamin, Diethylamin, Di-n-propylamin, Diisopropylamin, Di-n-butylamin, Pyrrolidin, Piperidin, Hexamethylenamin, Piperazin und Morpholin.

Bei den durch das erfindungsgemäße Verfahren hergestellten Alkylaminen oder Mischungen davon handelt es sich im allgemeinen um Alkylamine der Formeln 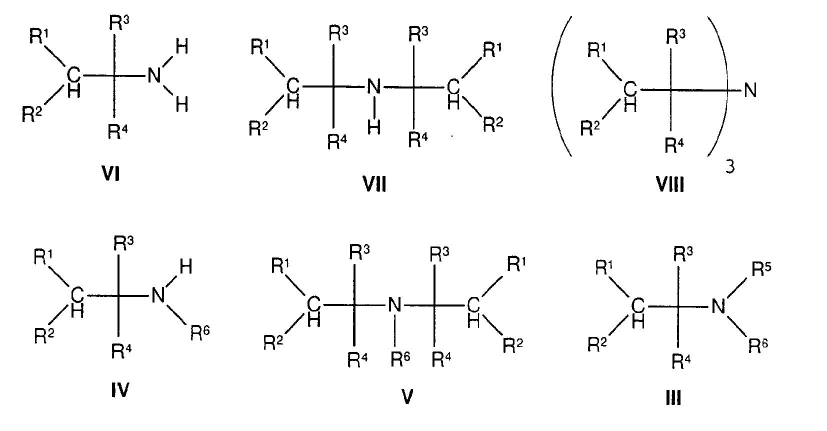 wobei R¹ bis R⁶ die oben genannten Bedeutungen besitzen.

Zur besseren Übersichtlichkeit des obigen Schemas wurden Regioisomere von **V**, **VII** und **VIII** (analog zu den in den noch folgenden Reaktionsschemata dargestellten Regioisomeren **Va** und **Vb**) nicht dargestellt, sind jedoch mit umfasst.

Falls man die Umalkylierung in der zweiten Verfahrensstufe an einem hydrier- oder dehydrieraktiven Katalysator in Gegenwart von H₂ durchführt, werden eventuelle ungesättigte Reste R¹ bis R⁶ gegebenenfalls im gleichen Verfahrensschritt in die entsprechenden gesättigten Reste überführt.

Beispiele für solche verfahrensgemäß hergestellte Alkylamine sind Monoethylamin, Diethylamin, Triethylamin, n-Propylamin, Di-n-propylamin, Tri-n-propylamin, iso-Propylamin, Diisopropylamin, n-Butylamin, Di-n-butylamin, Tri-n-butylamin, iso-Butylamin, tert.-Butylamin, n-Pentylamin, iso-Pentylamin, Cyclopentylamin, n-Hexylamin, Cyclohexylamin, n-Octylamin, n-Decylamin, 2-Phenylethylamin, N-Ethyl-pyrrolidin, N-Ethyl-piperidin, N-Ethyl-hexamethylenamin, N-Ethyl-piperazin und N-Ethyl-morpholin.

Das Verfahren lässt sich wie folgt ausführen:
Erste Verfahrensstufe:

In der ersten Verfahrensstufe wird ein Olefin, insbesondere ein Olefin der Formel I, mitAmmoniak, einemprimärenAminund/odereinemsekundären Amin, insbesondere Ammoniak, einem primären Amin und/oder sekundärem Amin der Formeln II, unter hydroaminierenden Bedingungen umgesetzt.

Vorteilhafterweise können dem Zulauf für die erste Verfahrensstufe auch Ammoniak und/oder Amine aus dem Reaktionsaustrag dieser ersten Verfahrensstufe und/oder aus der zweiten Verfahrensstufe zu(rück)geführt werden.

### a)

Insbesondere wird in der ersten Verfahrensstufe ein Olefin, insbesondere ein Olefin der Formel **I**, mit einem primären Amin und/oder einem sekundären Amin, insbesondere einem primären Amin und/oder sekundärem Amin der Formeln **II**, ganz besonders einem sekundärem Amin der Formel **II**, in Gegenwart eines Metallmonoalkylamids oder Metalldialkylamids oder Mischungen hiervon als Katalysator unter hydroaminierenden Bedingungen umgesetzt.

Die Umsetzung erfolgt im allgemeinen gemäß dem folgenden Reaktionsschema.

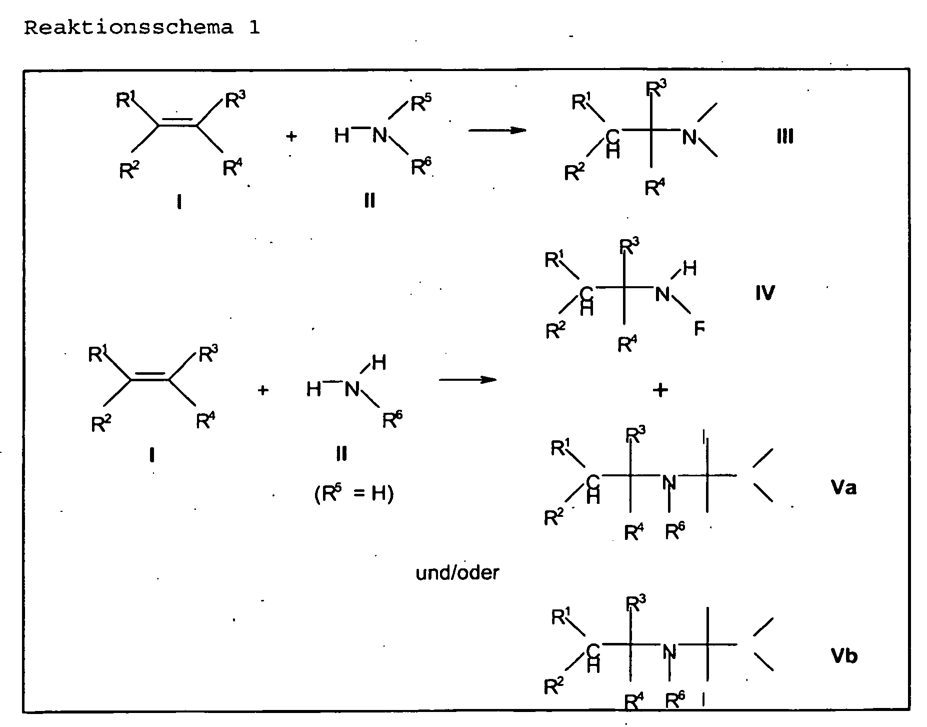

Vereinfachend für obiges Schema wurde angenommen, dass im Falle von ungesättigten Resten R¹ bis R⁴ in **I** die resultierende olefinische Doppelbindung weder in α- noch in β-Position zum Stickstoff in **III**, **IV** oder **V** zuliegenkommt. In Wirklichkeit kann jedoch unter den basischen Bedingungen in der ersten Verfahrensstufe durchaus Doppelbindungsisomerisierung auftreten.

Ganz besonders bevorzugte Olefine **I** sind Ethen (R¹, R², R³ und R⁴ = H), 1,3-Butadien (R¹, R³ und R⁴ = H und R² = CH=CH₂) und Propen (R¹, R² , R³ = H und R⁴ = CH₃).

Ganz besonders bevorzugte Amine sind Mono- und Dialkylamine wie Monoethylamin, Diethylamin, n-Butylamin, Di-n-Butylamin und Isopropylamin.

Hydroaminierungsprodukte ausgehend von Ethen und Monoethylamin sind Di- und/oder Triethylamin, ausgehen von Ethen und Diethylamin:
Triethylamin, ausgehend von Isopropylamin und Propen: Diisopropylamin, ausgehend von 1,3-Butadien und n-Butylamin: Butenyl-n-butylamine und ausgehend von Isopren und Isopentylamin: Isopentyl-methylbutenylamine.

Metalldialkylamide sind gegenüber Metallmonoalkylamiden als Katalysatoren bevorzugt.

Die Metallmonoalkylamide und Metalldialkylamide besitzen im allgemeinen die Formel MNR⁷R⁸ (M = einwertiges Metall), M(NR⁷R⁸)₂ (M = zweiwertiges Metall), M(NR⁷R⁸)₃ (M = dreiwertiges Metall) oder M(NR⁷R⁸)₄ (M = vierwertiges Metall), wobei

R⁷ die Bedeutungen gemäß dem Rest R⁵ und R⁸ die Bedeutungen gemäß dem Rest R⁶ hat und R⁷ und R⁸ auch gemeinsam eine gesättigte oder ungesättigte C₃- bis C₉-Alkylenkette, die durch ein O-, S- oder N-Heteroatom unterbrochen sein kann (wie für R⁵ und R⁶ definiert), insbesondere gemeinsam eine -(CH₂)ⱼ-X-(CH₂)ₖ- Gruppe (wie für R⁵ und R⁶ definiert), bedeuten kann (siehe oben).

Besonders bevorzugt besitzen die Metallmonoalkylamide und Metalldialkylamide die Formel MNR⁵R⁶, M (NR⁵R⁶)₂, M(NR⁵R⁶)₃ oder M(NR⁵R⁶)₄, in der demnach die Reste R denjenigen des eingesetzten primären und/oder sekundären Amins entsprechen.

Als Metallkomponente (M) in den Metallmonoalkylamid- und Metalldialkylamid-Katalysatoren sind Metalle der Gruppen IA, IIA, IIIB und IVB (Li, Na, K, Rb, Cs, Mg, Ca, Sr, Ba, Sc, Y, La oder ein Lanthanidelement, Ti, Zr oder Hf), insbesondere Li, Na, Mg, Ca und K, ganz besonders Na und K, bevorzugt.

Beispiele für solcheMetallalkylamide sindNaHNEt, NaNEt₂, KHNEt, KNEt₂, LiHNEt, LiNEt₂, LiN(iso-C₃H₇)₂, NaN(iso-C₃H₇)₂, NaN(n-C₄H₉)₂, NaHN(iso-C₃H₇), NaHN(iso-Pentyl), Mg(NEt₂)₂, Ca(NPr₂)₂ und Zr(NEt₂)₄.

Bei der Hydroaminierung von Ethen mit Monoethylamin und/oder Diethylamin sind NaNEt₂ und KNEt₂ besonders bevorzugt.

Bei der Hydroaminierung von Di-n-butylamin mit Butadien sind NaN(nBu)₂ und KN(nBu)₂, bei der von n-Butylamin mit Butadien NaHNnBu und KHNnBu besonders bevorzugt.

Die Herstellung dieser Metallmonoalkylamide und Metalldialkylamide erfolgt wie in Houben-Weyl, Methoden der organischen Chemie, 4. Aufl., BandXI/2 (Stickstoffverbindungen II und III), verlagThieme, Stuttgart, S. 182ff, in US-A-4,595,779, WO 93/14061, DE-A-21 17 970, DRP 615,468, GB-A-742 790, DE-A-26 13 113, US-A-2, 750, 417, J. Wollensak, Org. Synth. 43 (1963), Seite 45ff, oder C.A. Brown, J. Am. Chem. Soc. 95(3) (1973), Seite 982ff, beschrieben durch Umsetzung von Metall mit dem entsprechenden Amin in Gegenwart einer ungesättigten Verbindung wie z.B. Butadien, Isopren, Naphthalin, Pyridin oder Styrol, durch Umsetzung eines Metallamids oder -hydrids mit dem entsprechenden Amin oder durch Umsetzung einer Organometallverbindung, wie z. B. n-BuLi, MeLi, PhNa, Et₂Mg oder Et₄Zr, mit dem entsprechenden Amin.

Die Umsetzung des Olefins mit demAmin in Gegenwart des Metallalkylamids (erste Verfahrensstufe) kann z. B. wie in G. P. Pez et al . , Pure & Appl. Chem. 57(12), 1917-26 (1985), R.D. Closson et.al., J. Org. Chem. 22 (1957), 646-9, G.M. Whitman et al., US-A-2, 501,556), D. Steinborn et al., Z. Chem. 29 (1989), 333-4, D. Steinborn et al. , Z. Chem. 26 (1986) 349-59 und H. Lehmkuhl et al. , J. Organomet. Chem. 55 (1973), 215-20 beschrieben erfolgen.
Die Umsetzung des Olefins mit dem Amin in Gegenwart des Metallalkylamids kann auch in Gegenwart geringer Mengen von Ammoniak (kleiner 1 Mol% bezogen auf das/die eingesetzte/n Amine) durchgeführt werden (vergl. DE-A-21 17 970).

Die Herstellung des Metallalkylamids und die katalytische Hydroaminierung des Olefins kann auch in einer "Eintopfsynthese", d.h. simultan durchgeführt werden.

Man setzt dabei z. B. BuLi, C₂H₄ und Diethylamin oder Na, Diethylamin, C₂H₄ und wenig Butadien als Reaktionsmischung ein.

Besitzt das zu hydroaminierende Olefin konjugierte Doppelbindungen, wie z. B. Butadien, Isopren oder auch Styrol, so wird die Synthese bevorzugt als "Eintopfsynthese" ausgeführt.

Das Metallalkylamid kann während der Reaktion durch β-Eliminierung oder Einwirkung von H₂ wie in DE-A-26 13 113 beschrieben in Metallhydrid umgewandelt werden; im Falle der β-Eliminierung entsteht dabei parallel ein Imin. Dieses kann unter Einwirkung von primärem oder sekundärem Amin nach DE-A-26 13 113, C.A. Brown, J. Am. Chem. Soc. 95 (3) (1973), 982ff oder C.A. Brown, Synthesis (1978), 754ff, wieder in Metallalkylamid und H₂ umgewandelt werden, so dass das Metallhydrid als eine Art "Ruheform" des Metallalkylamids angesehen werden kann und daher im Sinne der vorliegenden Erfindung mit dem Metallalkylamid gleichzusetzen ist.

Weiter können Komplexbildner als Lösungsmittel sowohl bei der Katalysatorherstellung als auch bei der Reaktion zugegen sein.

Sobeschreiben z.B. J.F. Remenar (J. Am. Chem. Soc. 120 (1988), 4081ff), H. Lehmkuhl et al. (J. Organomet. Chem. 55 (1973), 215ff) und D. Steinborn et al. (Z. Chem. 29 (1989), 333ff) die Verwendung von N,N,N',N'-Tetramethylethylendiamin,
N,N,N',N",N"-Pentamethyldiethylentriamin,
N,N,N',N'-Tetramethylcyclohexandiamin und Tetrahydrofuran als Komplexbildner.

Des weiteren können Amine mit mehreren aminischen N-Atomen pro Molekül, wie z. B. N,N,N',N'-Tetraethylethylendiamin, N-permethyliertes bzw. N-perethyliertes Triethylentetramin bis hin zu N-permethyliertem bzw. N-perethyliertem Polyimin mit Molmassen bis 500.000 Dalton, Ether und Polyether,wiez.B.Diglyme,Triglyme und die entsprechenden Homologen, endgruppenverschlossene Polyole - z. B. PEG, PPG, Poly-THF -, und Komplexbildner mit aminischen N- und etherischen O-Atomen im Molekül, wie z. B. 3-Methoxyethylamin, 3-(2-Methoxyethoxy)propylamin oder N,N,N',N'-Tetramethyldiaminodiethylether, zugegen sein.

Man geht dabei in der Regel so vor, dass man den Katalysator (oder das entsprechende Metallhydrid oder die entsprechende Organometallverbindung (z. B. n-BuLi) als Katalysator 'vorläufer') in einem primären und/oder sekundären Amin, besonders bevorzugt in einem sekundären Amin, löst oder suspendiert. Bevorzugt verwendet man dabei als Lösungsmittel das Amingemisch welches sich aus dem Feed der ersten Verfahrensstufe und den Produkten der Hydroaminierung automatisch einstellt.

Der Katalysator kann als Lösung, als Suspension oder geträgert auf einem typischen Katalysatorträger wie z. B. SiO₂, Al₂O₃, TiO₂ ZrO₂, Aktivkohle, MgO, MgAl₂O₄ vorliegen. Bevorzugt liegt der Katalysator als Lösung oder Suspension, besonders bevorzugt als Lösung vor.

Die Hydroaminierung des Olefins kann diskontinuierlich, halbkontinuierlich oder kontinuierlich erfolgen.

Im ersten Fall wird das Olefin zu Katalysator und Amin gegeben und umgesetzt. Im zweiten Fall wird das Olefin zur Reaktionsmischung dosiert.

Im dritten Fall werden Katalysator, Amin und Olefin kontinuierlich zudosiert.

Bevorzugt ist ein molares Verhältnis Olefin:sekundärem Amin von 3:1 bis 1:10, besonders bevorzugt ist 1:1 bis 1:2.

Bevorzugt ist ein Verhältnis Olefin:primärem Amin von 6:1 bis 1:5, besonders bevorzugt ist 2:1 bis 1:1.

Die Reaktion findet bevorzugt unter Rühren bei 0 bis 250 °C, insbesondere bei 20 bis 150 °C und besonders bevorzugt bei 40 bis 120 °C statt.

Die Reaktion kann unter dem sich unter den gewählten Bedingungen ergebenden Druck (Eigendruck) durchgeführt werden. Im allgemeinen beträgt der Überdruck bei der Reaktionsdurchführung 0 bis 200 bar, insbesondere 2 bis 100 bar, ganz besonders 3 bis 30 bar.

Als Reaktoren kommen alle typischen Reaktionsapparate in Frage, z. B. Rührkessel, Schlaufenreaktoren, Blasensäulen, gepackte Blasensäulen, kaskadierte Blasensäulen und Rührkolonnen.

Nach der Reaktion wird das Produkt z. B. durch Destillation, Rektifikation, Filtration, Membranfiltration, Wasserwäsche oder Adsorption vom Katalysator getrennt.

Nichtprotolysierter Katalysator (Metallalkylamid oder Metallhydrid) kann anschließend zurückgeführt werden.

### b)

Insbesondere wird alternativ wird in der ersten Verfahrensstufe wird ein Olefin, insbesondere ein Olefin der Formel I, mit Ammoniak und/oder einem primären Amin, insbesondere Ammoniak und/oder einem primären Amin der Formel II, in Gegenwart einer anorganischen Festkörpersäure als Katalysator oder Mischungen hiervon unter hydroaminierenden Bedingungen umgesetzt.

Die Umsetzung erfolgt im allgemeinen gemäß dem folgenden Reaktionsschema.

### Reaktionsschema 2

vereinfachend wurde hier angenommen, dass im Falle von ungesättigten Resten R¹ bis R⁴ in I die resultierende olefinische Doppelbindung weder in α- noch in β-Position zum Stickstoff in **IV, V, VI, VII** oder **VIII** zu liegen kommt. In Wirklichkeit kann jedoch unter den sauren Bedingungen der ersten Verfahrensstufe durchaus Doppelbindungsisomerisierung auftreten.

Zur besseren Übersichtlichkeit des obigen Schemas wurden Regioisomere von **VII** und **VIII** analog zu den Regioisomeren **Va** und **Vb** nicht dargestellt, sind jedoch mit umfasst.

Ganz besonders bevorzugte Olefine **I** sind Ethen (R¹, R², R³ und R⁴ = H), Propen (R¹, R² und R⁴ = H und R³ - CH₃), 1- und 2-Buten (R¹, R² und R⁴ = H und R³= C₂H₅ bzw. R¹ und R³ = H und R² und R⁴ = CH₃), 1,3-Butadien (R¹, R³ und R⁴ = H und R² = CH=CH₂) und iso-Buten (R³ und R⁴ = CH₃, R¹ und R² = H).

Besonders bevorzugte Amine sind NH₃ und Monoalkylamine, besonders bevorzugt sind NH₃, Monoethylamin, n-Butylamin und Isopropylamin, ganz besonders bevorzugt ist NH₃.

Hydroaminierungsprodukte ausgehend von Ethen und Ammoniak sind hauptsächlich Mono-, wenig Di- und sehr wenig Triethylamin, ausgehend von Ethen und Monoethylamin: hauptsächlich Di- und wenig Triethylamin, ausgehend von iso-Buten und Ammoniak: tert.-Butylamin, ausgehend von 1,3-Butadien und Ammoniak: 1-Amino-2-buten und/oder 2-Amino-3-buten, ausgehend von Propen und Ammoniak: iso-Propylamin und ausgehend von Butenen und Ammoniak: 2-Butylamin.

Die als Katalysator eingesetzte anorganische Festkörpersäure ist dadurch definiert, dass sie
(1.) mehr als 50 ∝mol/g saure Zentren bei einem pKs-Wert kleiner 3,3 besitzt und
(2.) bis mindestens 400 °C thermisch stabil ist.

Die Zahl der sauren Zentren wird dabei nach der Methode der Hammett-Titration mit Dimethylgelb [CAS-No. 60-11-7] als Indikator und n-Butylamin als Sonde gemäß H.A. Benesi und B.H.C. Winquist in Adv. Catal., Vol. 27, Academic Press 1978, S. 100ff, bestimmt.

Beispiele für solche anorganischen Festkörpersäuren sind Zeolithe und Alumosilikate, Aluminiumphosphate bzw. Silica-Alumophosphate, gemischte saure Metalloxide und saure Metalloxide mit hoher Oberfläche, Schichtsilikate sowie geträgerte und ungeträgerte Ammoniumhalogenide.

Geeignete Katalysatoren für die Hydroaminierung von Olefinen mit Ammoniak und/oder einem primären Amin sind Zeolithe, insbesondere Faujasite wie X-, Y- und USY-Zeolith, Erionit, Chabazit, Mordenit, Offretit, Clinoptiolith, Pentasile wie ZSM- 5 und ZBM-10, ZSM-11, ZSM-12, MCM-22, MCM-41, MCM-48, MCM-49, MCM-56, EMT, SSZ-26, SSZ-33, SSZ-37, CIT-1, PSH-3, NU-85, BetasowiedieborhaltigenFormen, wie z.B. ZBM-11, H-Bor-ZSM-5, H-Bor-Beta, H-Bor-ZSM-11, sowie die gallium- oder titanhaltigen Formen. Sie zeichnen sich durch eine hohe Anzahl an katalytisch aktiven Zentren aus, kombiniert mit einer großen Oberfläche.

Die beschriebenen Zeolithe unterscheiden sich im Typ und in der Art der Nachbehandlung nach ihrer Herstellung (z. B. thermische Behandlung, Dealuminierung, Säurebehandlung, Metallioneneintausch, etc.).

Beispiele für geeignete Zeolithe finden sich in US-A-4,375,002, US-A-4,536,602, EP-A-305 564, EP-A-101 921 und DE-A-42 06 992.

Auch die aus EP-A-133 938, EP-A-431 451 und EP-A-132 736 bekannten Zeolithe, bei denen es sich um Bor-, Gallium-, Alumino- und Eisensilikatzeolithe handelt, die gegebenenfalls wie beschrieben mit Alkali-, Erdalkali- und Übergangsmetallen dotiert werden können, sind geeignet.

Weiterhinsindz. B. auch die aus CA-A-2 092 964 bekannten Beta-Zeolithe, die als kristalline Aluminosilikate bestimmter Zusammensetzung mit einer Porengröße von mehr als 5 Å definiert sind, geeignet.

Bevorzugt werden metall- oder halogenmodifizierte Beta-Zeolithe eingesetzt, wie z. B. in DE-A-195 30 177 beschrieben.

Insbesondere geeignet sind auch Zeolith-Katalysatoren des Pentasil-Typs mit einem SiO₂/Al₂O₃-Molverhältnis von größer/gleich 10, wie sie in EP-A-132 736 offenbart sind.

Unter die Aluminiumphosphate und Silico-Alumophosphate fallen die kristallinen Systeme mit Zeolithstrukturen oder zeolithähnlichen Strukturen wie z. B. SAPO-37, AlPO₄-5, SAPO-5, wie in DE-A-196 01 409 beschrieben, aber auch amorphe Systeme wie sie z. B. in DE-A-44 31 093 beschrieben sind. Sie besitzen allgemein die Formel Al₂O₃*P₂O₅*xSiO₂.

Als saure gemischte Metalloxide kommen zudem die Systeme in Frage, die Tanabe et al. in Bull. Chem. Soc. Jpn. 47 (1974), S. 1064ff, beschreibt, insbesondere Al₂O₃-TiO₂, SiO₂-TiO₂, ZrO₂-TiO₂, Fe₂O₃-TiO₂, WO₃-TiO₂, MoO₃-TiO₂, Nb₂O₅-TiO₂, Al₂O₃-B₂O₃, SiO₂-Ga₂O₃, SiO₂-B₂O₃, SiO₂-Y₂O₃, SiO₂-ZrO₂, SiO₂-WO₃, SiO₂-MoO₃, ZrO₂-MoO₃, ZrO₂-WO₃.

Beispiele für die Aminierung von Olefinen mit diesen Oxiden finden sich in DE-A-196 24 206.

Die als Katalysator einsetzbaren Schichtsilikate sind insbesondere Tone der Montmorillonit-Saponit-, der Kaolin-Serpentin- und der Palygorskit-Sepiolith-Gruppe, z.B. Montmorillonit, Hectorit, Kaolin, Attapulgit, Sepiolith, Beidellit, Montronit, Saponit, Sauconit, wie sie in Klockmanns Lehrbuch der Mineralogie, 16. Aufl., F. Euke Verlag (1978), Seiten 739-765, beschrieben sind. Zusätzlich können diese Schichtsilikate noch modifiziert sein, z. B. durch das sogenannte "pillaring" (die PILC' s) oder durcheine Säureaktivierung (z. B. Tonsil, K10 und K20 der Fa. Südchemie AG, München).

Beispiele zur Herstellung von Aminen aus Olefinen und NH₃ mit Schichtsilikaten finden sich in DE-A-195 24 242.

Des weiteren können diese sauren Katalysatoren auch bereits gebrauchtes Material beinhalten oder aus solchem Material bestehen, welches durch die üblichen Methoden regeneriert wurde, z. B. durcheineRekalzinierung in Luft, H₂O, CO₂ oder Inertgas bei Temperaturen größer 200 °C, durch Waschen mit H₂O, Säuren oder organischen Lösungsmitteln, durch Steamen oder durch Behandlung im Vakuum bei Temperaturen größer 200 °C.

Sie können in Form von Pulver oder bevorzugt in Form von Formkörpern wie Strängen, Tabletten oder Splitt eingesetzt werden. Für die Verformung kann 2 bis 60 Gew. -% (bezogen auf die zu verformende Masse) Bindemittel zugesetzt werden. Als Bindemittel eignen sich verschiedene Aluminiumoxide, bevorzugt Boehmit, amorphe Aluminosilikate mit einem molaren SiO₂/Al₂O₃-Verhältnis von 25:75 bis 95:5, Siliciumdioxid, bevorzugt hochdisperses SiO₂ wie z.B. Silikasole, Gemische aus hochdispersem SiO₂ und hochdispersem Al₂O₃, hochdisperses TiO₂ sowie Tone.

Nach der Verformung werden die Extrudate oder Presslinge zweckmäßig bei 110 °C/16 h getrocknet und bei 300 bis 500 °C/2 bis 16 h calciniert, wobei die Calcinierung auch direkt im Hydroaminierungsreaktor erfolgen kann.

In der Regel werden die Katalysatoren in der H-Form eingesetzt. Zur Erhöhung der Selektivität, der Standzeit und der Anzahl der möglichen Katalysatorregenerierungen kann man jedoch zudem verschiedene Modifizierungen an den Katalysatoren vornehmen.

Eine Modifizierung der Katalysatoren besteht darin, dass man die unverformten Katalysatoren mit Alkalimetallen wie Na und K, Erdalkalimetallen wie Ca, Mg, Erdmetallen wie T1, Übergangsmetallen wiebeispielsweiseMn, Fe, Mo, Cu, Zn, Cr, Edelmetallenund/oderseltenen Erdmetallen wie z.B. La, Ce oder Y ionenaustauschen bzw. dotieren kann.

Eine vorteilhafte Katalysatorausführungsform besteht darin, daß man die verformten Katalysatoren in einem Strömungsrohr vorlegt und bei 20 bis 100 °C z. B. ein Halogenid, ein Acetat, ein Oxalat, ein Citrat oder ein Nitrat der oben beschriebenen Metalle in gelöster Form darüberleitet. Ein derartiger Ionenaustausch kann z. B. an der Wasserstoff-, Ammonium- und Alkaliform der Katalysatoren vorgenommen werden.

Eine andere Möglichkeit der Metallaufbringung auf die Katalysatoren besteht darin, dass man das zeolithische Material z. B. mit einem Halogenid, Acetat, Oxalat, Citrat, Nitrat oder Oxid der oben beschriebenen Metalle in wässriger oder alkoholischer Lösung imprägniert.

Sowohl an einen Ionenaustausch als auch an eine Imprägnierung kann man eine Trocknung, wahlweise eine abermalige Calcination anschließen. Bei den metalldotierten Katalysatoren kann eine Nachbehandlung mit Wasserstoff und/oder mit Wasserdampf günstig sein.

Eine weitere Möglichkeit der Modifizierung des Katalysators besteht darin, dass man das heterogenkatalytische Material - verformt oder unverformt - einer Behandlung mit Säuren, wie Salzsäure (HCl), Flusssäure (HF), Phosphorsäure (H₃PO₄), Schwefelsäure (H₂SO₄) Oxalsäure (HO₂C-CO₂H) oder deren Gemische unterwirft.

Eine besondere Ausführungsform besteht darin, daß man das Katalysatorpulver vor seiner Verformung mit Flusssäure (0,001 bis 2 molar, bevorzugt 0,05 bis 0, 5 molar) 1 bis 3 Stunden unter Rückfluss behandelt. Nach Abfiltrieren und Auswaschen wird in der Regel bei 100 bis 160 °C getrocknet und bei 400 bis 550 °C calciniert.

Eine weitere besondere Ausführungsform liegt in einer HCl-Behandlung der Heterogenkatalysatoren nach ihrer Verformung mit Bindemittel. Hierbei wird der Heterogenkatalysator in der Regel 1 bis 3 Stunden bei Temperaturen zwischen 60 und 80 °C mit einer 3 bis 25 %igen, insbesondere mit einer 12 bis 20 %igenSalzsäure behandelt, anschließend ausgewaschen, bei 100 bis 160 °C getrocknet und bei 400 bis 550 °C calciniert.

Eine andere Möglichkeit der Modifizierung des Katalysators ist der Austausch mit Ammoniumsalzen, z. B. mit NH₄Cl, oder mit Mono-, Di- oder Polyaminen. Hierbei wird der mit Bindemittel verformte Heterogenkatalysator in der Regel bei 60 bis 80 °C mit 10 bis 25 %iger, bevorzugt ca. 20 %iger, NH₄Cl-Lösung 2 h kontinuierlich in gewichtsmäßiger Heterogenkatalysator/Ammoniumchlorid-Lösung von 1:15 ausgetauscht und danach bei 100 bis 120 °C getrocknet.

Eine weitere Modifikation, die an aluminiumhaltigen Katalysatoren vorgenommen werden kann, ist eine Dealuminierung, bei der ein Teil der Aluminiumatome durch Silicium ersetzt wird oder die Katalysatoren durch beispielsweise hydrothermale Behandlunginihrem Aluminiumgehalt abgereichert werden. An eine hydrothermale Dealuminierung schließt sich vorteilhafterweise eine Extraktion mit Säuren oder Komplexbildnern an, um gebildetes Nichtgitteraluminium zu entfernen. Der Ersatz von Aluminium durch Silicium kann beispielsweise mit Hilfe von (NH₄)₂SiF₆ oder SiCl₄ erfolgen. Beispiele für Dealuminierungen von Y-Zeolithen finden sich in Corma et al., Stud. Surf. Sci. Catal. 37 (1987), Seiten 495 bis 503.

Die Katalysatoren kann man als Stränge mit Durchmessern von z. B. 1 bis 4 mm oder als Tabletten mit z. B. 3 bis 5 mm Durchmesser für die Hydroaminierung der Olefine einsetzen.

Die Umsetzung des Olefins mit Ammoniak und/oder dem primären Amin in Gegenwart der anorganischen Festkörpersäure kann z. B. wie in EP-A-132 736, EP-A-752 409 und EP-A-822 179 beschrieben erfolgen.

Man geht dabei in der Regel so vor, dass man Ammoniak und/oder primäres Amin zusammen mit Olefin in einem molaren Verhältnis von 1: 1 bis 10:1, bevorzugt 1:1 bis 5:1, mischt und in einem Festbettreaktor oder in einer Wirbelschicht bei einem Druck von 40 bis 700 bar, bevorzugt 200 bis 300 bar, und einer Temperatur von 80 bis 400 °C, bevorzugt 250 bis 350 °C, in der Gasphase oder im überkritischen Zustand umsetzt.

Alternativ kann die Reaktion in der Flüssigphase bei einem Druck von 40 bis 80 bar und einer Temperatur von 60 bis 120 °C in einem Rührkessel, einem Fest-Flüssig-Fließbett oder einem Strömungsrohr durchgeführt werden.

Eine Ausführungsform dieses Verfahrens besteht darin, dass man Ammoniak und/oder das primäre Amin zusammen mit dem Olefin oder dem Olefingemisch im molaren Verhältnis von 1:1 bis 5: 1 gemischt einem Festbettreaktor, der die anorganische Festkörpersäure enthält, zuführt und bei einem Druck von 100 bis 300 bar, bevorzugt 120 bis 300 bar, insbesondere 140 bis 290 bar, und einer Temperatur von 200 bis 350 °C; bevorzugt 220 bis 330 °C, insbesondere 230 bis 320 °C, in der Gasphase oder im überkritischen Zustand umsetzt.

Die Lage des Gleichgewichts und damit der Umsatz zum gewünschten Hydroaminierungsprodukt ist stark vom gewählten Reaktionsdruck abhängig. Hoher Druck begünstig das Additionsprodukt, doch stellt im allgemeinen aus technischen und wirtschaftlichen Gründen der Druckbereich bis 300 bar das Optimum dar. Die Selektivität der Reaktion wird - neben Größen wie Ammoniak-/Amin-Überschuss und Katalysator - in hohem Maß durch die Temperatur beeinflußt. Zwar nimmt die Reaktionsgeschwindigkeit der Additionsreaktion mit steigender Temperatur stark zu, doch werden gegebenenfalls selektivitätsmindernde Nebenreaktionen gleichzeitig gefördert. Zudem ist eine Temperaturerhöhung aus thermodynamischer Sicht meist nicht vorteilhaft. Die Lage des Temperaturoptimums bezüglich Umsatz und Selektivität ist von der Konstitution des Olefins, des eingesetzten primären Amins und des Katalysators abhängig und liegt meist im Bereich von 200 bis 350°C.

### c)

In einer weiteren besonderen Alternative wird in der ersten Verfahrensstufe ein Olefin, insbesondere ein Olefin der Formel I, mit Ammoniak, einem primären Amin und/oder einem sekundären Amin, insbesondere Ammoniak, einem primären Amin und/oder einem sekundären Amin der Formeln II, in Gegenwart einer
Übergangsmetallkomplexverbindung als Katalysator oder Mischungen hiervon unter hydroaminierenden Bedingungen umgesetzt.

Die Umsetzung erfolgt im allgemeinen gemäß den obigen Reaktionsschemata 1 und 2 und den jeweiligen Anmerkungen zu diesen Schemata.

Ganz besonders bevorzugte Olefine **I** sind Ethen, (R¹, R², R³ und R⁴ = H), Propen (R¹, R² und R³ = H und R⁴ = CH₃) und 1, 3-Butadien (R¹, R³ und R⁴ = H und R² =CH=CH₂).

Neben Ammoniak sind ganz besonders bevorzugte Amine Mono- und Dialkylamine wie Monomethylamin, Dimethylamin, Monoethylamin, Diethylamin, n-Butylamin, Isopropylamin, Diisopropylamin und Di-n-butylamin.

Hydroaminierungsprodukte ausgehend von Ethen und Ammoniak sind Mono-, Di- und/oder Triethylamin, ausgehend von Ethen und Monoethylamin: Di-und/oder Triethylamin, ausgehend von iso-Buten und Ammoniak: tert.-Butylamin, ausgehend von 1,3-Butadien'und Ammoniak: 1-Amino-3-buten und/oder 2-Amino-3-buten, ausgehend von 1,3-Butadien und n-Butylamin: (2-Butenyl)-n-butylamin und/oder (3-Butenyl) -n-butylamin und ausgehend von Propylen und Isopropylamin: Diisopropylamin.

Bei der als Katalysator eingesetzten Übergangsmetallkomplexverbindung handelt es sich im allgemeinen um eine Komplexverbindung eines Metalls der Gruppe IIIB, IVB oder VIII des Periodensystems.

Beispiele hierfür sind die in US-A-3,758,586 beschriebenen Verbindungen des Rhodiums oder Iridiums, wie (Cyclooctadien)₂RhCl₂, RhCl₃, RhBr₃ und IrCl₃, für die Addition von sekundären aliphatischen Aminen an Ethylen zu aliphatischen tertiären Aminen,
die in US-A-4,454,321 beschriebenen Verbindungen des Rutheniums und Eisens, wie RuCl₃ * xH₂O, Ru (cyclopentadienyl) ₂, [Ru(NH₃)₄(OH)Cl]Cl * 2H₂O, Fe(CO)₅, Fe₂(CO)₉, H₂Fe(CO)₄, Fe(butadien)(CO)₃, Fe(CO)₅/ Tri-n-butylphosphin, Fe(CO)₅/Triphenylphosphit, für die Addition von Ammoniak, primärer Amine oder sekundärer Amine an Olefine, wie z. B. Ethen,
die im Übersichtsärtikel von Th.E. Müller und M. Beller in Chem. Rev. 1998, Vol. 98, No. 2, Seiten 675 bis 703, auf den Seiten 679 bis 680 zitierten Verbindungen des Platins und Palladiums, wie PtX₄²⁻ (X = Cl⁻, Br-), für die Hydroaminierung von Olefinen,
die im o. g. Übersichtsartikel von Th.E. Müller und M. Beller auf den Seiten 681 bis 684 zitierten Verbindungen des Lanthans, Neodymiums, Samariums und Lutetiums, wie Cp*₂Ln-E (Cp* = Pentamethylcyclopentadienyl oder Tetramethylcyclopentadienyl mit Brücke zum zweiten Tetramethylcyclopentadienylligand, Ln = La, Nd, Sm oder Lu, E = H, CH(SiMe₃)₂ oder N(SiMe₃)₂), für die Hydroaminierung (insbesondere die intramolekulare Hydroaminierung) von Olefinen und
die im o. g. Übersichtsartikel von Th.E. Müller und M. Beller auf den Seiten 684 bis 686 zitierten Verbindungen des Platins, Rutheniums, Hafniums, Zirkoniums, Iridiums und Tantals, wie Pt(PEt₃)₂(H)(NHPh), IrCl(C₂H₄)₂(PEt₃)₂ und Cp₂Zr(H)[N(t-Bu)(SiHMe₂)], für die Hydroaminierung von Olefinen.

Die Umsetzung des Olefins mit dem Ammoniak und/oder dem Amin kann z. B. wie in der oben genannten Literatur beschrieben erfolgen.

Die Reaktion kann kontinuierlich, in Batch-Fahrweise oder in Semi-Batch-Fahrweise betrieben werden.

Bei den Batch-Fahrweisen wird der Katalysator zusammen mit dem Amin vorgelegt. Nach Erreichen der Reaktionstemperatur wird das Olefin aufgepresst. Nach Absinken des Drucks (Maß für die Reaktion) wird das Produkt bzw. Produktgemisch abdestilliert.

Überschüssiges Olefin sowie nicht abreagiertes Amin können zurückgeführt werden.

Bei der Batch-Fahrweise kann der Katalysator zusammen mit dem Produktgemisch über Sumpf aus dem Reaktor gefahren werden und separat aufgearbeitet werden.

Die Reaktion kann in einem Rührkessel durchgeführt werden.

Bei kontinuierlicher Fahrweise kann die Reaktion in einer Blasensäule durchgeführt werden. Hierbei wird das Olefin von unten durch das Gemisch aus Katalysator und Produktlösung geblasen. Die Lösung kann dann destillativ vom Katalysator entfernt oder der Katalysator kann mittels Membran aus der Produktlösung entfernt werden. Alternativ braucht der Katalystor nicht entfernt zu werden, sondern kann direkt in die Aufarbeitung bzw. in den nächsten Verfahrensschritt gegeben werden, sofern er dort nicht stört.

### Zweite Verfahrensstufe:

Das oder die in der ersten Verfahrensstufe erhaltene/n Hydroaminierungsprodukt/e wird/werden anschließend in einer zweiten Verfahrensstufe unter umalkylierenden Bedingungen umgesetzt.

Erfindungsgemäß wird/werden das oder die in der ersten Verfahrensstufe erhaltene/n Hydroaminierungsprodukt/e anschließend in der zweiten Verfahrensstufe
d) in Gegenwart eines Umalkylierungskatalysators oder,
e) in Gegenwart von Wasserstoff und eines umalkylierenden Hydrier-oder Dehydrierkatalysators
bei Temperaturen von 80 bis 400 °C umgesetzt.

Der Reaktionsaustrag aus der ersten Verfahrensstufe enthält Hydroaminierungsprodukte, entsprechend des/der jeweils eingesetzten Olefins/Olefine und Amins/Amine und der jeweils gewählten hydroaminierenden Bedingungen (siehe oben). Diese Hydroaminierungsprodukte weisen im allgemeinen die Formeln III, IV, V, VI, VII und/oder VIII (siehe obige Reaktionsschemata 1 und 2 und die dazugehörigen Anmerkungen) auf.

Der Reaktionsaustrag aus der ersten Verfahrensstufe kann, in der Regel nach Abtrennung des Katalysators (wie jeweils oben beschrieben), direkt in die zweite Verfahrensstufe eingesetzt werden.

Aus dem Reaktionsaustrag der ersten Verfahrensstufe kann/können jedoch auch zunächst das oder die für die zweite Verfahrensstufe gewünschte/n Hydroaminierungsprodukt/e (wie jeweils oben beschrieben) abgetrennt und danach in die zweite Verfahrensstufe eingesetzt werden.

Vorteilhafterweise können dem Zulauf für die zweite Verfahrensstufe auch Ammoniak und/oder Amine zugeführt, und/oder Ammoniak und/oder Amine aus dem Reaktionsaustrag dieser zweiten Verfahrensstufe zu(rück)geführt werden.

Die Umsetzung des/der in der ersten Verfahrensstufe erhaltenen Hydroaminierungsprodukts/-produkte in der zweiten Verfahrensstufe unter umalkylierenden Bedingungen kann z. B. wie in Houben Weyl Band XI/1, Stickstoffverbindungen II, 1957, Georg Thieme Verlag Stuttgart, S. 248 - 261, beschrieben erfolgen.

Demnach kann die Aminumalkylierung ('Amintausch') in Gegenwart von Umalkylierungskatalysatoren wie Säuren, Zeolithen (wie ab Seite 14, Zeile 4, beschrieben), Metallsalzen, Iod, Dehydratationskatalysatoren, Hydrier-/Dehydrierkatalysatoren oder auch in Abwesenheit von Katalysatoren durchgeführt werden.

Als Llmalkylierungskätalysator geeignete Säuren sind z. B. Halogenwasserstoffsäuren (wie HCl), Phosphorsäure, Sulfanilsäure oder Sulfonsäuren (wie p-Toluolsulfonsäure).

Als Umalkylierungskatalysator geeignete Metallsalze sind z. B. Zink-oder Eisenhalogenide, wie Zinkchlorid, Eisen (II) chlorid oder Eisen(III)chlorid.

Als Umalkylierungskatalysator geeignete Dehydratationskatalysatoren sind z. B. Mangan(II)oxid/Aktivkohle, Aluminiumsilikate, Al₂O₃, TiO₂ oder ZrO₂.

Als Umalkylierungskatalysator bevorzugt sind Hydrier- und Dehydrierkatalysatoren.

Dabei ist zur Aufrechterhaltung der katalytischen Aktivität die Anwesenheit von Wasserstoff vorteilhaft. Alternativ kann der Hydrier-oder Dehydrierkatalysator in regelmäßigen Abständen mit H₂ reduktiv von Belägen befreit werden.

Als Hydrier- und Dehydrierkatalysatoren sind Katalysatoren, die als katalytisch aktive Bestandteile Elemente, ausgewählt aus der Gruppe Kupfer, Silber, Gold, Eisen, Cobalt, Nickel, Rhenium, Ruthenium, Rhodium, Palladium, Osmium, Iridium, Platin, Chrom, Molybdän und Wolfram, jeweils in metallischer Form (Oxidationsstufe 0) oder in Form von Verbindungen wie z. B. Oxiden, die unter den Verfahrensbedingungen zum entsprechenden Metall reduziert werden, enthalten, besonders geeignet.

Die katalytisch aktiven Bestandteile Kupfer, Silber, Gold, Eisen, Cobalt, Nickel, Rhenium, Ruthenium, Rhodium, Palladium, Osmium, Iridium, Platin, Chrom, Molybdän und/oder Wolfram sind im allgemeinen insgesamt in Mengen von 0, 1 bis 80 Gew. -%, bevorzugt 0, 1 bis 70 Gew. -%, besonders bevorzugt 0,1 bis 60 Gew.-%, berechnet als Metall in der Oxidationsstufe 0, in der katalytisch aktiven Masse des Katalysators enthalten.

Bevorzugt sind Katalysatoren, die als katalytisch aktive Bestandteile Elemente, ausgewählt aus der Gruppe Kupfer, Silber, Cobalt, Nickel, Ruthenium, Rhodium, Palladium, Platin, Chrom und Molybdän, insbesondere ausgewählt aus der Gruppe Kupfer, Cobalt, Nickel, jeweils in metallischer Form (Oxidationsstufe 0) oder in Form von Verbindungen wie z. B. Oxiden, die unter den Verfahrensbedingungen zum entsprechenden Metall reduziert werden, enthalten.

Mehr bevorzugt sind Katalysatoren, die die katalytisch aktiven Bestandteile Kupfer, Silber, Eisen,Cobalt,Nickel, Ruthenium, Rhodium, Palladium, Osmium, Iridium und/oder Platin und ein Trägermaterial, bevorzugt ausgewählt aus der Gruppe Aluminiumoxid, Zirkoniumdioxid, Titandioxid, Kohlenstoff und/oder sauerstoffhaltige Verbindungen des Siliziums, enthalten.

Die katalytisch aktive Masse dieser im erfindungsgemäßen Verfahren bevorzugt eingesetzten Katalysatoren enthält die katalytisch aktiven Bestandteile Kupfer, Silber, Eisen, Cobalt,Nickel, Ruthenium,Rhodium, Palladium, Osmium, Iridium und/oder Platin im allgemeinen insgesamt in Mengen von 0,1 bis 80 Gew.-%, bevorzugt 0,1 bis 70 Gew.-%, besonders bevorzugt 0, 1 bis 60 Gew. -%, berechnet als Metall in der Oxidationsstufe 0.

Weiterhin enthält die katalytisch aktive Masse dieser bevorzugt eingesetzten Katalysatoren die Trägermaterialien Aluminiumoxid (Al₂O₃), Zirkoniumdioxid (ZrO₂), Titandioxid (TiO₂), Kohlenstoff und/oder sauerstoffhaltige Verbindungen des Siliziums, berechnet als SiO₂, im allgemeinen insgesamt in Mengen von 20 bis 99,9 Gew.-%, bevorzugt 30 bis 99,9 Gew.-%, besonders bevorzugt 40 bis 99,9 Gew.-%.

Besonders bevorzugt sind Katalysatoren mit den Aktivkomponenten Cu, Co, Ni und/oder Pd, insbesondere Cu, Co und/oder Ni. Diese können als Vollkontakte oder als Trägerkatalysatoren verwendet werden

Ganz besonders bevorzugt werden Cu-haltige Katalysatoren, die, wie erfindungsgemäß erkannt wurde, wegen ihrer vergleichsweise geringen Ethan- bzw. Methanbildung selektiver sind.

Beispiele hierfür sind Kupferlegierungen, metallisches Kupfer, z. B. in Form von Kupfernetz, und Cu-Katalysatoren mit einem Cu-Gehalt von 2 bis 70 Gew.-% Cu, berechnet als CuO, auf einem Träger, bevorzugt mit 10 bis 55 Gew.-% Cu, berechnet als CuO, auf einem Träger. Trägermaterialien können bevorzugt Aluminiumoxid (Al₂O₃), Zirkoniumdioxid (ZrO₂), Titandioxid (TiO₂) Kohlenstoff und/oder sauerstoffhaltige Verbindungen des Siliziums sein.

Beispielsweise können in der EP-A-382 049 offenbarte Katalysatoren, deren katalytisch aktive Masse vor der Behandlung mit Wasserstoff 20 bis 85 Gew.-%, bevorzugt 70 bis 80 Gew.-%, ZrO₂, 1 bis 30 Gew.-%, bevorzugt 1 bis 10 Gew.-%, CuO, und jeweils 1 bis 40 Gew.-%, bevorzugt 5 bis 20 Gew.-%, CoO und NiO enthält, beispielsweise die in loc. cit. auf Seite 6 beschriebenen Katalysatoren mit der Zusammensetzung 76 Gew. -% Zr, berechnet als ZrO₂, 4 Gew.-% Cu, berechnet als CuO, 10 Gew.-% Co, berechnet als CoO, und 10 Gew.-% Ni, berechnet als NiO, im erfindungsgemäßen Verfahren eingesetzt werden.

Weiterhin können im erfindungsgemäßen Verfahren die in der EP-A-963 975 offenbarten Katalysatoren, deren katalytisch aktive Masse vor der Behandlung mit Wasserstoff
22 bis 40 Gew.-% ZrO₂,
1 bis 30 Gew. -% sauerstoffhaltige Verbindungen des Kupfers, berechnet als CuO,
15 bis 50 Gew.-%sauerstoffhaltige Verbindungen des Nickels, berechnet als NiO, wobei das molare Ni : Cu-Verhältnis größer 1 ist,
15 bis 50 Gew. -% sauerstoffhaltige Verbindungen des Kobalts, berechnet als CoO,
0 bis 10 Gew.-% sauerstoffhaltige Verbindungen des Aluminiums und/oder Mangans, berechnet als Al₂O₃ bzw. MnO₂,
   und keine sauerstoffhaltigen Verbindungen des Molybdäns enthält, beispielsweise der in loc. cit., Seite 17, offenbarte Katalysator A mit der Zusammensetzung 33 Gew.-% Zr, berechnet als ZrO₂, 28 Gew.-% Ni, berechnet als NiO, 11 Gew.-% Cu, berechnet als CuO und 28 Gew.-% Co, berechnet als CoO, eingesetzt werden.

Weiterhin können im erfindungsgemäßen Verfahren in der EP-A-514 692 offenbarte Katalysatoren, deren katalytisch aktive Masse vor der Behandlung mit Wasserstoff 5 bis 100 Gew.-% eines Oxides von Kupfer und Nickel im Atomverhältnis von 1 : 1 bis 10 : 1, bevorzugt von 2 : 1 bis 5 : 1, und Zirkon- und/oder Aluminiumoxid enthält, insbesondere die in loc. cit. auf Seite 3, Zeilen20bis30, offenbartenKatalysatoren, deren katalytisch aktive Masse vor der Behandlung mit Wasserstoff 20 bis 80, besonders 40 bis 70, Gew. - % Al₂O₃ und/oder ZrO₂, 1 bis 30 Gew. -% CuO, 1 bis 30 Gew. - % NiO und 1 bis 30 Gew. - % CoO enthält, eingesetzt werden.

Bevorzugt können in der DE-A-19 53 263 offenbarte Katalysatoren enthaltend Kobalt, Nickel und Kupfer und Aluminiumoxid und/oder Siliciumdioxid mit einem Metallgehalt von 5 bis 80 Gew. -%, insbesondere 10 bis 30 Gew.-%, bezogen auf den gesamten Katalysator, wobei die Katalysatoren, berechnet auf den Metallgehalt, 70 bis 95 Gew.-% einer Mischung aus Kobalt und Nickel und 5 bis 30 Gew.-% Kupfer enthalten und wobei das Gewichtsverhältnis von Kobalt zu Nickel 4 : 1 bis 1 : 4, insbesondere 2 : 1 bis 1 : 2, beträgt,
die in EP-A-696 572 offenbarten Katalysatoren, deren katalytisch aktive Masse vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ bzw. MnO₂, enthält, beispielsweise der in loc. cit., Seite 8, offenbarte Katalysator mit der Zusammensetzung 31, 5 Gew.-% ZrO₂, 50 Gew.-% NiO, 17 Gew.-% CuO und 1,5 Gew.-% MoO₃,
die in EP-A-284 919 offenbarten Katalysatoren der allgemeinen Formel MₓMg_{y}(SiO₂)·nH₂O, worin M ein zweiwertiges, reduzierbares Metallatom aus der Gruppe Cu, Fe, Co und Ni, x und y Zahlen sind, die zusammen den Wert 1,5 erreichen können, und n nach Trocknung ausgedrückt in Gew. -% zwischen 0 und 80 liegt, beispielsweise der in loc. cit imBeispiel beschriebene Katalysator enthaltend 35 % CuO, 9 % MgO und 38 % SiO₂ und der in EP-A-863 140 auf Seite 3 beschriebene Katalysator enthaltend 45 bis 47 Gew.-% CuO,
Magnesiumsilikat aus etwa 15 bis 17 Gew.-% MgO und 35 bis 36 Gew.-% SiO₂, etwa 0, 9 Gew.-% Cr₂O₃, etwa 1 Gew.-% BaO und etwa 0,6 Gew.-% ZnO,
die in DE-A-24 45 303 offenbarten Katalysatoren, die durch Temperung eines basischen Kupfer und Aluminium enthaltenen Carbonats der allgemeinen Zusammensetzung CuₘAl₆(CO₃)_{0,5m}O₃(OH)ₘ₊₁₂, wobei m einen beliebigen, auch nicht ganzzahligen, Wert zwischen 2 und 6 bedeutet, bei einer Temperatur von 350 bis 700 °C erhältlich sind, beispielsweise der in loc. cit., Beispiel 1, offenbarte kupferhaltige Fällkatalysator, der durch Behandlung einer Lösung von Kupfernitrat und Aluminiumnitrat mit Natriumbicarbonat und anschließendem Waschen, Trocknen und Tempern des Präzipitats hergestellt wird, und
die in WO 95/32171 und EP-A-816 350 offenbarten Trägerkatalysatoren enthaltend 5 bis 50, bevorzugt 15 bis 40, Gew.-% Kupfer, berechnet als CuO, 50 bis 95, bevorzugt 60 bis 85, Gew.-% Silicium, berechnet als SiO₂, 0 bis 20 Gew.-% Magnesium, berechnet als MgO, 0 bis 5 Gew.-% Barium, berechnet als BaO, 0 bis 5 Gew.-% Zink, berechnet als ZnO, und 0 bis 5 Gew.-% Chrom, berechnet als Cr₂O₃, jeweils bezogen auf das Gesamtgewicht des calcinierten Katalysators, beispielsweise der in EP-A-816 350, Seite 5, offenbarte Katalysator enthaltend 30 Gew.-% CuO und 70 Gew.-% SiO₂,
   erfindungsgemäß eingesetzt werden.

Die im erfindungsgemäßen Verfahren als Umalkylierungskatalysator verwendete Hydrier- oder Dehydrierkatalysatoren können nach den im Stand der Technik beschriebenen Verfahren hergestellt und teilweise auch kommerziell erhalten werden.

Bei der Herstellung von Trägerkatalysatoren existieren bezüglich der Aufbringungsmethode der Aktivkomponenten, wie z. B. Nickel, Cobalt und/oder Kupfer und gegebenenfalls weitere Komponenten, auf das verwendete Trägermaterial keinerlei Beschränkungen.

Insbesondere kommen folgende Aufbringungsmethoden in Betracht:
a) Tränkung
   Aufbringung eine Metallsalzlösung in einer oder mehreren Tränkstufen auf einen vorgefertigten anorganischen Träger. Der Träger wird im Anschluß an die Tränkung getrocknet und ggf. calciniert.
a1) Die Tränkung kann nach der sogenannten "incipient wetness"-Methode erfolgen, bei der der Träger entsprechend seiner Wasseraufnahmekapazität maximal bis zur Sättigung mit der Tränklösung befeuchtet wird. Die Tränkung kann aber auch in überstehender Lösung erfolgen.
a2) Bei mehrstufigen Tränkverfahren ist es zweckmäßig, zwischen einzelnen Tränkschritten zu trocknen und ggf. zu calcinieren. Die mehrstufige Tränkung ist vorteilhaft besonders dann anzuwenden, wenn der Träger mit einer größeren Metallmenge beaufschlagt werden soll.
a3) Bevorzugt wird das anorganische Trägermaterial bei der Tränkung als vorgeformte Masse eingesetzt, beispielsweise als Pulver, Kugeln, Stränge oder Tabletten. Besonders bevorzugt wird der Einsatz als Pulver.
a4) Als Lösungsmittel der Metallsalze wird bevorzugt konzentrierter wässriger Ammoniak eingesetzt.
a5) Die Einbringung von Promotoren kann in einem Schritt analog a1) durch Tränkung mit einer entsprechend metallhaltigen Tränklösung, z. B. kupfer-, cobalt- und/oder nickelhaltigen Tränklösung, und promotorhaltigen Tränklösung oder mehrstufig analog a2) durch wechselweise Tränkung mit metallhaltiger Tränklösung und promotorhaltiger Tränklösung erfolgen.
b) Fällung
   Fällung einer Metallsalzlösung auf einen vorgefertigten, inerten anorganischen Träger. Dieser liegt in einer besonders bevorzugten Ausführungsform als Pulver in einer wässrigen Suspension vor.
b1) IneinerAusführungsform (i) wirdeineMetallsalzlösung, bevorzugt mit Sodalösung, gefällt. Als Vorlage wird eine wässrige Suspension des Trägermaterials verwendet.
b2) In einer weiteren Ausführungsform (ii) kann der Fällkatalysator in einem Zwei-Stufen-Prozess hergestellt werden. Dabei wird in einer ersten Stufe ein Pulver gemäß den Angaben aus a) hergestellt und getrocknet. Dieses Pulver wird in eine wäßrige Suspension überführt und als Vorlage äquivalent zu der in Ausführungsform (i) beschriebenen eingesetzt.
b3) Die Einbringung von Promotoren kann in einem Schritt analog b1) durch Fällung einer metallhaltigen Lösung oder mehrstufig analog b2) durch sukzessive Fällung einer metallhaltigen Lösung und promotorhaltiger Lösung erfolgen. Im letztgenannten Fall können die einzelnen Fällungen direkt aufeinander folgen oder durch einen Waschprozess und/oder Trocknungsprozess und/oder Kalzinierprozess getrennt sein.

Als Ausgangssubstanzen für a) und/oder b) können prinzipiell alle in den bei der Aufbringung verwendeten Lösungsmitteln löslichen Metall (I)-und/oder Metall (II) -Salze, beispielsweise Sulfate, Nitrate, Chloride, Carbonate, Acetate, Oxalate oder Ammonium-Komplexe, verwendet werden. Besonders bevorzugt für Verfahren gemäß a) werden Metallcarbonate eingesetzt, für Verfahren gemäß b) Metallnitrate.

Ausgefällte Niederschläge, die aus a) oder b) resultieren, werden in üblicher Weise filtriert und vorzugsweise alkalifrei gewaschen.

Es ist auch möglich, in den filtrierten und gegebenenfalls gewaschenen Niederschlag eine Promotorkomponente in geeigneter Form einzubringen. Geeignete Formen sind beispielsweise anorganische Salze oder Komplexe oder organische Verbindungen.

Sowohl die Endprodukte aus a) als auch die aus b) werden bei Temperaturen von 50 bis 150 °C, vorzugsweise bei 100 bis 140°C, getrocknet und gegebenenfalls im Anschluss, z. B. über einen Zeitraum von 2 Stunden, bei höherer Temperatur, d. h. im allgemeinen 200 bis 400 °C, insbesondere bei 200 bis 220 °C, getempert.

Es ist sowohl nach der Trocknung als auch nach der Temperung möglich, eine Promotorkomponente in geeigneter Form einzubringen. Geeignete Formen sind beispielsweise anorganische Salze oder Komplexe oder organische Verbindungen. Die Einbringung erfolgt dabei zweckmäßig durch intensives Mischen, Kneten und/oder Verdichten, wobei gegebenfalls auch Flüssigkeiten, wie beispielsweise Wasser oder Alkohole zugegeben werden können. Nach Einbringung der Promotorkomponente erfolgtzweckmäßigerweise ein weiterer Trocknungs-und/oder Temperugnsschritt. Bei Zugabe im trockenen Zustand kann dieser jedoch auch gegebenenfalls entfallen.

Für die Verwendung im erfindungsgemäßen Verfahren wird das oben beschriebene getrocknete Pulver bevorzugt zu Tabletten oder ähnlichen Formkörpern verformt. Als Tablettierungshilfsmittel wird für den verformungsprozeß Graphit, vorzugsweise in einem Anteil von 3 Gew. -%, bezogen auf das Gewicht des getrockneten Pulvers, zugegeben.

Die Tablettenformkörper werden, vorzugsweise 2 Stunden, bei 300 bis 600 °C, insbesondere bei 330 bis 350 °C getempert. Dieses besondere Verfahren zur Tablettierung erlaubt, im Vergleich zum ausschließlichen Einsatz von Graphit als Tablettierungshilfsmittel in den üblichen Verfahren, eine besonders leicht durchzuführende Verformung des Pulvers zu Tabletten und liefert sehr chemisch und mechanisch stabile Katalysatoren.

Es ist auch möglich, in die geformten Tabletten eine Promotorkomponente in geeigneter Form einzubringen. Geeignete Formen sind beispielsweise Lösungen anorganischer Salze oder Komplexe oder organische Verbindungen. Nach Einbringung erfolgt zweckmäßigerweise eine erneute Trocknung bei Temperaturen von 50 bis 150 °C, vorzugsweise 100 bis 140°C. Zusätzlich kann noch eine Temperung, vorzugsweise für ca. 2 Stunden, bei 300 bis 600 °C, insbesondere bei 330 bis 350 °C erfolgen.

Erfolgt die Umsetzung in der zweiten Verfahrensstufe unter umalkylierenden und gleichzeitig hydrierenden Bedingungen, insbesondere in Gegenwart von Wasserstoff und einem umalkylierenden Hydrier-oder Dehydrierkatalysator, können im Hydroaminierungsaustrag aus der ersten Verfahrensstufe enthaltene ungesättigte organische Sticktoffverbindungen, d. h. organische Stickstoffverbindungen, die entweder eine olefinische C=C-Doppelbindung (z. B. wie in Enaminen und Allylaminen) aufweisen oder Imine darstellen, gegebenenfalls zu den entsprechend gesättigten Aminen hydriert werden.

Als umalkylierender Hydrier- oder Dehydrierkatalysator werden hierbei die gleichen Hydrier- und Dehydrierkatalysatoren wie bereits oben beschrieben bevorzugt.

Der Hydroaminierungsaustrag aus der ersten Verfahrensstufe enthält z. B. ungesättigte organische Sticktoffverbindungen, die eine olefinische C=C-Doppelbindung aufweisen, wenn in der ersten Verfahrensstufe als Olefin ein zwei- oder mehrfaches Olefin eingesetzt wurde oder wenn in der ersten Verfahrensstufe als Amin ein entsprechend ungesättigtes Amin eingesetzt wurde. Imine können durch Doppelbindungsisomerisierung oder durch β-Eliminierung aus Metallalkylamiden entstehen.

Beispiele für solche zwei- oder mehrfache Olefine sind: 1,3-Butadien, 1,4-Pentadien, 1,5-hexadien, Allen, Isopren, 4-Vinyl-1-cyclohexen, Cyclohexadien.

Beispiele für solche ungesättigte Amine mit einer olefinischen C=C-Doppelbindung sind:Allylamin, 1-Amino-2-buten, 1-Amino-3-buten, 1-Amino-4-penten.

Zur Durchführung der zweiten Verfahrensstufe geht man bevorzugt so vor, dass das Produkt- bzw Produktgemisch aus der ersten Reaktionsstufe, oder das aus der Aufarbeitung der ersten Verfahrensstufe stammende Amin bzw. angereicherte Amin kontinuierlich über einen Umalkylierungskatalysator gefahren wird oder diskontinuierlich umalkyliert wird.

Bei einer kontinuierlichen Fahrweise wird der Umalkylierungskatalysator in einen Rohrreaktor oder Rohrbündelreaktor eingebaut.
Im Falle eines umalkylierenden Dehydrier-/Hydrierreaktors und der Fahrweise in Gegenwart von H₂ (Variantee) kann der Katalysator wahlweise vorher mit Wasserstoff reduziert werden, er kann aber auch in Gegenwart des Produktes und Wasserstoff direkt angefahren werden.

Der Wasserstoffdruck kann zwischen 0 bar und 300 bar, bevorzugt zwischen 1 und 250 bar, gewählt werden.

Bei einer Umsetzung in der Gasphase liegt der Druck im allgemeinen bei 1 bis 70 bar.

Bei einer Umsetzung in der Flüssigphase liegt der Druck im allgemeinen bei 70 bis 250 bar.

Die Temperatur liegt im allgemeinen bei 80 bis 400 °C, insbesondere zwischen 100 und 350 °C, bevorzugt zwischen 120 und 250 °C, ganz besonders bevorzugt zwischen 150 und 230 °C.

Je nach gewählter Temperatur stellt sich ein thermodynamisches Gleichgewicht der verfahrensgemäßen Alkylamine plus ggf. Ammoniak ein, das von dem Verhältnis Stickstoff zu Alkylgruppen abhängt. Je sterisch anspruchsvoller die Alkylgruppen sind, desto geringer wird der Anteil des entprechenden tertiären Alkylamins.

Die Belastung des Katalysators mit dem Startmaterial kann zwischen 0, 05 und 2 kg Startmaterial pro Liter Katalysator undpro Stunde (kg/l*h), bevorzugt zwischen 0,1 und 1 kg/l*h, besonders bevorzugt zwischen 0,2 und 0,6 kg/l*h, betragen.

Das molare Verhältnis der Amine (der Umalkylierungspartner') (in den bevorzugten Fällen Ammoniak oder/und primäres Amin) kann je nach gewünschtem Produktmix in weiten Bereichen variieren.

Im Falle einer diskontinuierlichen Umalkylierung kann der Katalysator zusammen mit dem Startmaterial für die zweite Verfahrensstufe vorgelegt und die gewünschte Menge des Umalkylierungspartners dazugegeben oder ergänzt werden. Anschließend wird auf die gewünschte Temperatur erwärmt (s.o.).

Nach Druckentspannung kann der Austrag aufdestilliert werden.

Eine ganz besonders bevorzugte Ausführungsform des erfindungsgemäßen Verfahrens ist die Umsetzung von Ethen mit Monoethylamin und/oder bevorzugt Diethylamin (erste Verfahrensstufe) und anschließende Umsetzung der erhaltenen Ethylamine (insbesondere Triethylamin) mit Ammoniak (zweite Verfahrensstufe) und Rückführung von überschüssigem Ammoniak in die zweite Verfahrensstufe und teilweiser Rückführung von Monoethylamin und/oder bevorzugt Diethylamin in die erste Verfahrensstufe. Die gewünschten Ethylamine als Produkte des erfindungsgemäßen Verfahrens werden, ggf. unter Rückführung überschüssiger Ethylamine in die erste oder/und zweite Verfahrensstufe, in beliebigem Mengenverhältnis zueinander nach Aufarbeitung des Reaktionsaustrages der ersten oder/und der zweiten Verfahrensstufe erhalten.

Eine weitere ganz besonders bevorzugte Ausführungsform des erfindungsgemäßen Verfahrens ist die Umsetzung von Propen mit Monoisopropylamin (erste Verfahrensstufe) und anschließende Umsetzung des erhaltenen Diisopropylamins mit Ammoniak (zweite Verfahrensstufe) und Rückführung von überschüssigem Ammoniak in die zweite Verfahrensstufe und teilweiser Rückführung von Monoisopropylamin in die erste Verfahrensstufe. Die gewünschten Isopropylamine als Produkte des erfindungsgemäßen Verfahrens werden, ggf. unter Rückführung überschüssiger Isopropylamine in die erste oder/und zweite Verfahrensstufe, in beliebigem Mengenverhältnis zueinander nach Aufarbeitung des Reaktionsaustrages der ersten oder/und der zweiten Verfahrensstufe erhalten.

Noch eine ganz besonders bevorzugte Ausführungsform des erfindungsgemäßen Verfahrens ist die Umsetzung von Butadien mit n-Monobutylamin und/oder bevorzugt n-Dibutylamin (erste Verfahrensstufe) und anschließende Umsetzung der erhaltenen n-Butylamine (insbesondere n-Tributylamin) mit Ammoniak (zweite Verfahrensstufe) und Rückführung von überschüssigem Ammoniak in die zweite Verfahrensstufe und teilweiser Rückführung von n-Monobutylamin und/oder bevorzugt n-Dibutylamin in die erste Verfahrensstufe. Die gewünschtenn-Butylamine werden, ggf. unter Rückführung überschüssiger n-Butylamine in die erste oder/und zweite Verfahrensstufe, in beliebigem Mengenverhältnis zueinander nach Aufarbeitung des Reaktionsaustrages der ersten oder/und der zweiten Verfahrensstufe erhalten.

Das erfindungsgemäße Verfahren wird im Weiteren ausführlich am Beispiel der Herstellung der Ethylamine beschrieben. Die Beschreibung gilt analog auch für die n-Butylamine und Isopropylamine und auch für andere Alkylamine. Die angegebenen Zusammensetzungen der Ströme können bezüglich ihrer molaren Zusammensetzung auf die Herstellung der n-Butylamine und Isopropylamine sinngemäß übertragen werden.

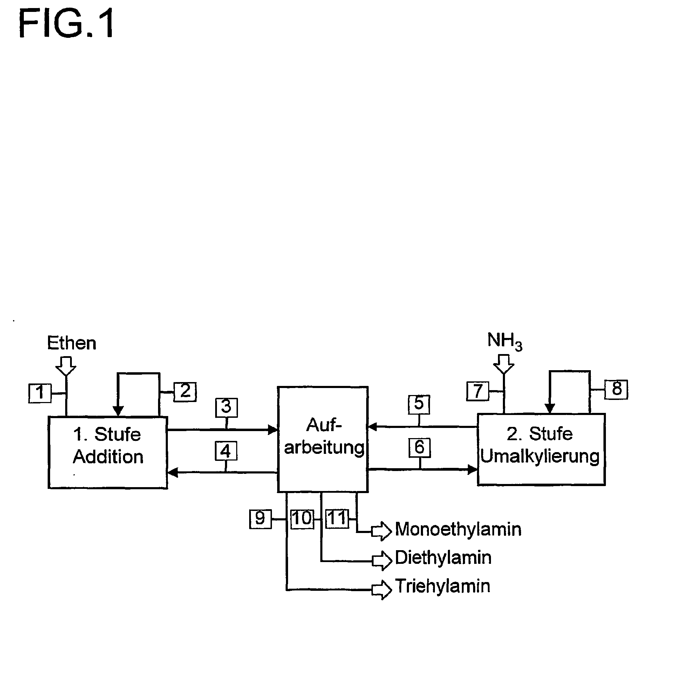

Schematisch werden diese bevorzugten Verfahren mit der Verschaltung der Stoffströme auf dem im Anhang befindlichen Blockfließbild (Abbildung 1) am Beispiel der Umsetzung von Ethen mit Ammoniak dargestellt.

In der ersten Verfahrensstufe wird (z. B. gemäß Beispiel 4) Ethen mit Diethylamin und/oder Monoethylamin, bevorzugt mit Diethylamin, in Gegenwart katalytischer Mengen eines Alkalimetalldiethylamids (Herstellung des Alkalimetalldiethylamidsz.B.gemäß Beispiel 1) unter hydroaminierenden Bedingungen umgesetzt.

Die dem Reaktor kontinuierlich zugeführten Ströme (1; 2; 4) bestehen insgesamt aus 0 - 1Gew.-%, bevorzugt < 0,1 Gew.-%, Ammoniak, 0-5 Gew.-%, bevorzugt < 1 Gew.-%, Monoethylamin, 20 - 80 Ges.-%, bevorzugt 40 - 70 Gew.-%, Diethylamin, 0 - 50 Gew.-%, bevorzugt < 40 Ges.-%, Triethylamin, 5 - 0 Gew.-%, bevorzugt 10 - 30 Gew.-%, Ethylen, 0,01 - 10 Gew.-% des Katalysators, vorzugsweise ein Alkalimetalldialkylamid, und 0-20 Gew.-% eines Lösungsmittels für den Katalysator. Als Lösungsmittel sind die bereits oben in der Rubrik a) Genannten geeignet.

In einem diskontinuierlichen Verfahren entsprechen diese Stromangaben den Startkonzentrationen im Reaktor.

Die Reaktion kann in diversen Reaktoren, z. B. in einer Blasensäule (bevorzugt kaskadiert), einem Rührkessel, einem Strahlschlaufenreaktor oder einer Reaktorkaskade, in der Regel bei 40 bis 150 °C und 1 bis 100 bar, insbesondere bei 70 bis 120 °C und 3 bis 20 bar, durchgeführt werden.

Der Katalysator liegt homogen gelöst in der Flüssigphase vor. Prinzipiell kann der Reaktor bei überschreiten der Löslichkeit des Katalysators auch in Suspensionsfahrweise betrieben werden.

Bei einer diskontinuierlichen Auslegung des Verfahrens werden die gebildeten Additionsprodukte (Hydroaminierungsprodukte) aus dem Reaktor abdestiliert. Der Katalysator, vorzugsweise ein Alkalimetalldialkylamid, kann - solange er über eine ausreichende Aktivität verfügt - im Reaktor verbleiben und kann so für weitere Umsetzungen genutzt werden.

Bei der kontinuierlichen Auslegung des Verfahrens kann das gebildete Addukt (Triethylamin) z. B. durch strippen mit unumgesetzten Ethen der Reaktionsmischung entzogen werden. Die Reaktionsmischung kann auch einer Flashverdampfung oder einer Destillation zugeführt werden, wobei der Katalysator, der in einem hochsiedendem Lösungsmittel (> 50 Gew.-%) oder in Trialkylamin (> 50 Gew.-%) gelöst oder suspendiert ist, am Sumpf anfällt. Der katalysatorhaltige Sumpf wird dem Reaktor der ersten Verfahrensstufe zurückgeführt. Ein Teilstrom wird zur Hochsieder- und Katalysatorausschleusung entsorgt. Alternativ zur thermischen Aufarbeitung des Reaktionsaustrages kann zur Katalysatorrückführung bzw. Rückhaltung z. B. eine Filtration (Nanofiltration, Membranfiltration, usw.) eingesetzt werden.

Der zu 30 bis 100 %, bevorzugt zu 80 - 100 %, aus Triethylamin bestehende Destillationsaustrag (Strom 3) wird in der zweiten Verfahrensstufe (z.B. gemäß Beispiel 7 oder 8) mit Ammoniak (Ammoniakeinspeisung: Strom 7) in Gegenwart von Wasserstoff an einem Umalkylierungskatalysator (z. B. dem im Beispiel 7 oder Beispiel 8 genannten Katalysator) heterogenkatalytisch bei 1 bis 250 bar und 100 bis 300 °C, insbesondere bei 180 bis 250 °C und 40 bis 200 bar, zu einem Gemisch von Mono- (5- 70, bevorzugt 10- 30 Ges.-%), Di- (20 - 80, bevorzugt, 30 - 60 Gew.-%), Triethylamin (5 - 70, bevorzugt 15- 50 Ges.-%) und Ammoniak (1- 70, bevorzugt 2- 10 Gew.-%) umgesetzt. Der Wasserstoffanteil beträgt 0,1 - 50, bevorzugt 0,5 - 5 Gew.-%.

(Der Reaktoraustrittsstrom ergibt sich insgesamt aus Strom 5 und 8, der Reaktoreintrittsstrom besteht aus Mono- (0 - 50, bevorzugt kleiner 30 Gew.-%), Di- (0 - 60, bevorzugt kleiner 30 Gew.-%), Triethylamin (10 - 95, bevorzugt 30 - 80 Ges.-%) und Ammoniak (1 - 70, bevorzugt 3-50 Gew.-%). Der Wasserstoffänteil beträgt 0,1- 50, bevorzugt 0, 5 - 5 Ges.-%).

Geeignet als Reaktoren sind Wirbel-, Fließ-, und Festbettreaktoren vorzugsweise jedoch Festbettrektoren. Die Reaktion kann zweiphasig sowohl als fest-flüssig als auch fest-gasförmig sowie dreiphasig betrieben werden.

Der Reaktionsaustrag wird anschließend, um die Zielprodukte Mono-, Di- sowie Triethylamin zu erhalten, mit den üblichen verfahrenstechnischen Methoden aufgearbeitet. So kann der heiße Reaktionsaustrag z. B. mittels Quench oder Kondensator abgekühlt und die Zielprodukte auskondensiert werden. Die verbleibende Gasphase bestehend aus Ammoniak, Wasserstoff sowie entsprechend ihren Partialdrücken auch die jeweiligen Amine wird auf Reaktionsdruck verdichtet und dem Reaktor der 2. Stufe zurückgeführt. Das Kondensat bzw. der flüssige Quenchaustrag (Strom 5) werden in einer geeigneten Destillationssequenz zu den Wertprodukten (Strom 9; 10; 11) (siehe Abbildung 1) entsprechend der gewünschten Spezifikation aufgearbeitet. Im Kondensat gelöster Ammoniak bzw. Wasserstoff werden abgetrennt und dem Reaktor inklusiv der für das gewünschte Mengenverhältnis der drei Ethylamine notwendigen Mengen Monoethylamin und Triethylamin zurückgeführt (Strom 6). Fällt Ammoniak bzw. Wasserstoff gasförmig an, so können diese verdichtet oder vorzugsweise mit dem Destillatstrom aus der 1. Stufe absorbiert werden. DerDestillatstromwirdanschließend bei Reaktionsdruck und Temperatur verdampft und dem Reaktor der 2. Stufe zugeführt.

Das am Reaktoraustritt der 2. Verfahrensstufe gewünschte Mengenverhältnis der drei Ethylamine zueinander kann in weiten Grenzen durch Verweilzeitvariation und durch das Triethylamin/Ammoniak-Gewichtsverhältnis, das zwischen 0,1-50 und bevorzugt zwischen 0,5-20 liegt, im Zulauf der zweiten Verfahrensstufe eingestelltwerden. DurchRückführungvonDiethylaminindie 1. oder/und 2. Stufe, vorzugsweise natürlich in die 1. Stufe zur Umsetzung mit Ethylen zu Triethylamin und Rückführung von Monoethylamin und/oder Triethylamin in die 1. oder/und 2. Stufe, vorzugsweise jedoch in die 2. Stufe, können die Amine in beliebigem Mengenverhältnis zueinander hergestellt werden. Inklusive der rückgeführten Mengen ergibt sich die oben beschriebene Zusammensetzung des Reaktoreingangsstroms (1. Stufe).

Die Reste R¹ bis R⁷ in den Formeln I bis VIII bedeuten unabhängig voneinander:
R¹, R², R³, R⁴:
Wasserstoff (H),
C₁- bis C₂₀-Alkyl, bevorzugt C₁- bis C₁₂-Alkyl, besonders bevorzugt C₁-bis C₈-Alkyl, wie Methyl, Ethyl, n-Propyl, iso-Propyl, n-Butyl, iso-Butyl, sec.-Butyl,tert.-Butyl,n-Pentyl,iso-Pentyl, neo-Pentyl, n-Hexyl, iso-Hexyl, n-Heptyl, iso-Heptyl, n-Octyl und iso-Octyl,
C₂- bis C₂₀-Alkenyl, bevorzugt C₂- bis C₁₂-Alkenyl, besonders bevorzugt C₂- bis C₈-Alkenyl, wie Vinyl und Allyl,
C₃- bis C₂₀-Cycloalkyl, bevorzugt C₃- bis C₁₂-Cycloalkyl, besonders bevorzugt C₅- bis C₈-Cycloalkyl, wie Cyclopentyl, Cyclohexyl, Cycloheptyl und Cyclooctyl,
C₅- bis C₈-Cycloalkenyl, wie 2-Cyclopent-1-yl, 2-Cyclohex-1-yl, 3-Cyclohex-1-yl,
C₆- bis C₂₀-Alkylcycloalkyl, bevorzugt C₄- bis C₁₂-Alkylcycloalkyl, besonders bevorzugt C₅- bis C₁₀-Alkylcycloalkyl, wie 2-Methyl-cyclopentyl und 4-Methyl-cyclohexyl,
C₆- bis C₂₀-Cycloalkyl-alkyl, bevorzugt C₄- bis C₁₂-cycloalkyl-alkyl, besonders bevorzugt C₅- bis C₁₀-Cycloalkyl-alkyl, wie Cyclopentylmethyl und Cyclohexylmethyl,
Aryl, wie Phenyl, 1-Naphthyl und 2-Naphthyl, bevorzugt Phenyl,
C₇- bis C₂₀-Alkylaryl, bevorzugt C₇- bis C₁₆-Alkylaryl, bevorzugt C₇- bis C₁₂-Alkylphenyl, wie 2-Methylphenyl, 3-Methylphenyl, 4-Methylphenyl, 2-Ethylphenyl, 3-Ethylphenyl und 4-Ethylphenyl,
C₇- bis C₂₀-Aralkyl, bevorzugt C₇- bis C₁₆-Aralkyl, bevorzugt C₇- bis C₁₂-Phenalkyl, wie Phenylmethyl, 1-Phenylethyl, 2-Phenylethyl,
R¹ und R³:
Gemeinsam eine C₂- bis C₁₂-Alkylenkette, bevorzugt eine C₃- bis C₈-Alkylenkette, besonders bevorzugt (CH₂)₃-, (CH₂) ₄-, (CH₂)₅-, -(CH₂)₆- und - (CH₂)₇-, insbesondere -(CH₂)₃- und - (CH₂)₄-,
R⁵:
Wasserstoff (H),
R⁵, R⁶:
C₁- bis C₂₀-Alkyl, C₂- bis C₂₀-Alkenyl, C₃- bis C₂₀-Cycloalkyl, jeweils wie oben für R¹ bis R⁴ definiert,
Aralkyl, insbesondere C₇- bis C₂₀-Aralkyl, wie oben für R¹ bis R⁴ definiert,
Alkoxyalkyl, insbesondere C₂₋₃₀-Alkoxyalkyl, bevorzugt C₂₋₂₀-Alkoxyalkyl, besonders bevorzugt C₂₋₈-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxy-ethyl und 2-Methoxyethyl, besonders bevorzugt C₂- bis C₄-Alkoxyalkyl,
Aminoalkyl, insbesondere C₁₋₂₀-Aminoalkyl, bevorzugt C₁₋₈-Aminoalkyl, wie Aminomethyl, 2-Aminoethyl, 2-Amino-1,1-dimethylethyl, 2-Amino-n-propyl, 3-Amino-n-propyl, 4-Amino-n-butyl, 5-Amino-n-pentyl, N-(2-Aminoethyl)-2-aminoethyl und N-(2-Aminoethyl)aminomethyl,
Monoalkylaminoalkyl, insbesondere C₂₋₃₀-Monoalkylaminoalkyl, bevorzugt C₂₋₂₀-Monoalkylaminoalkyl, besonders bevorzugt C₂₋₈-Monoalkylaminoalkyl, wie Methylaminomethyl, 2-Methylaminoethyl, Ethylaminomethyl, 2-Ethylaminoethyl und 2-iso-Propylaminoethyl,
Dialkylaminoalkyl, insbesondere C₃₋₃₀-Dialkylaminoalkyl, bevorzugt C₃₋₂₀-Dialkylaminoalkyl, besonders bevorzugt C₃₋₁₀-Dialkylaminoalkyl, wie N,N-Dimethylaminomethyl, (N,N-Dibutylamino)methyl, 2-(N,N-Dimethylamino)ethyl, 2-(N,N-Diethylamino)ethyl, 2-(N,N-Dibutylamino)ethyl, 2-(N,N-Di-n-propylamino)ethyl und 2-(N,N-Di-iso-propylamino)ethyl,
R⁵ und R⁶:
Gemeinsam eine gesättigte oder ungesättigte C₃- bis C₉-Alkylenkette, die durch ein O-, S- oder N-Heteroatom unterbrochen sein kann, wie z.B. -(CH₂)₃-, -(CH₂)₄-, -(CH₂)₅-, -(CH₂)₅-, -(CH₂)₆- und -CH=CH-CH=CH-,
insbesondere gemeinsam eine -(CH₂)ⱼ-X-(CH₂)ₖ- Gruppe, mit j und k unabhängig voneinander = 1, 2, 3 oder 4 und X = CH₂, CHR⁷, Sauerstoff (O), Schwefel (S) oder NR⁷,
   wobei R⁷=Hoder C₁- bis C₄-Alkyl, wieMethyl, Ethyl, n-Propyl, iso-Propyl, n-Butyl, iso-Butyl sec.-Butyl, tert.-Butyl,
   wie z. B. -(CH₂)₂-NH-(CH₂)₂-, -(CH₂)₂-NCH₃-(CH₂)₂-, -(CH₂)₂-O-(CH₂)-.

### Beispiele

### Beispiel 1

### Herstellung von Natriumdiethylamid und Kaliumdiethylamid

NaNEt₂ und KNEt₂ wurden in Anlehnung an WO 93/14061 wie folgt hergestellt: 100 mmol Na bzw. K wurden unter Argon in 30 ml absolutem, siedendem Toluol dispergiert. Dann wurde die Suspension abgekühlt und bei 20°C langsam eine über 3 Å-Molsieb getrocknete Mischung aus 140 mmol Isopren, 140 mmol Diethylamin und 35 ml absolutem Toluol zugetropft. Während einer Nachrührzeit von 1 h bildete sich schließlich NaNEt₂ bzw. KNEt₂. Dieses wurde unter Argon von der Lösung abzentrifugiert und mittels 19,1 g über 3 Ä-Molsieb getrocknetem HNEt₂ in einen 100 ml-Stahlautoklaven überführt.

### Beispiel 2

### Herstellung von Lithiumdiethylamid

In einem 250 ml Glaskolben wurden unter Argon 140 mmol über 3 Å-Molsieb getrocknetes Diethylamin vorgelegt und langsam bei 25 °C 100 mmol BuLi (2, 5M Lösung in Hexan) zugetropft, wobei sich eine Suspension von LiNEt₂ bildete. Durch Zugabe von weiteren 260 mmol Diethylamin resultierte eine homogene Lösung, welche in einen 100 ml-Stahlautoklaven überführt wurde.

### Beispiel 3

### Herstellung von Natriumdi-n-butylamid und Umsetzung von Di-n-butylamin mit Butadien in Gegenwart von Natriumdi-n-butylamid

NaNBu₂ wurde in Anlehnung an WO 93/14061 wie folgt hergestellt: In einem 250 ml Glaskolben wurden unter Argon 50 mmol Na bei 110 °C in 30 ml abs. n-Octan dispergiert. Nach Erkalten wurde eine Mischung bestehend aus 60 mmol Isopren, 60 mmol über 3 Ä-Molsieb getrocknetem Di-n-Butylamin und 30 ml abs. n-Octan zugetropft und 1 h nachgerührt. Das gebildete Natriumdi-n-butylamid wurde unter Argon abzentrifugiert, in 640 mmol Di-n-Butylamin gelöst und in einen 270 ml Stahlautoklaven überführt. Dieser wurde auf 80°C erwärmt und unter Rühren wurden binnen 5 h 475 mmol Butadien eingepreßt. Nach einer Nachrührzeit von 1h wurde der Autoklav abgekühlt, geöffnet und der Inhalt mit 25 ml 10 Gew.-% KCl in H₂O behandelt. Der Versuch wurde noch zweimal wiederholt, alle organischen Phasen vereinigt, mit 3Å-Molsiebgetrocknet und hydrierend isomerisiert (s. u. Beispiel 9).-

### Beispiel 4

### Umsetzung von Diethylamin mit Ethen in Gegenwart von Natriumdiethylamid als Katalysator

In den Autoklaven aus Beispiel 1 wurde bei Raumtemperatur 40 ml Diethylamin zugepumpt. Nach Erwärmen auf 90 °C wurde Ethen in geringem Unterschuß (7,2 g) zugeführt und solange gerührt, bis der Überdruck von anfänglich 18 bar auf 0 bar absank (7 h). Das_Produktgemisch (Diethylamin/Triethylamin) wurde aus demAutoklaven herausdestilliert (Siedetemperatur: 105 °C) . Diese Prozedur wurde 15 mal wiederholt (29 turnover (TO)), ohne eine Desaktivierung zu erkennen.

### Beispiel 5

### Umsetzung von Diethylamin mit Ethen in Gegenwart von Natriumdiethylamid als Katalysator

Die Katalysatorherstellung wurde wie in Beispiel 1 durchgeführt. Dabei wurden nur 50 mmol Katalysator verwendet. Nach Erwärmen auf 90 °C wurde Ethen in geringem Unterschuß (7,3 g) zugeführt und solange gerührt, bis der Überdruck von anfänglich 19 bar auf 2 bar absank (7 h). Das Produktgemisch (Diethylamin/Triethylamin) wurde aus dem Autoklaven herausdestilliert (Siedetemperatur: 102 °C) . Diese Prozedur wurde 5 mal wiederholt (20 turnover (TO) ), ohne eineDesaktivierung zu erkennen.

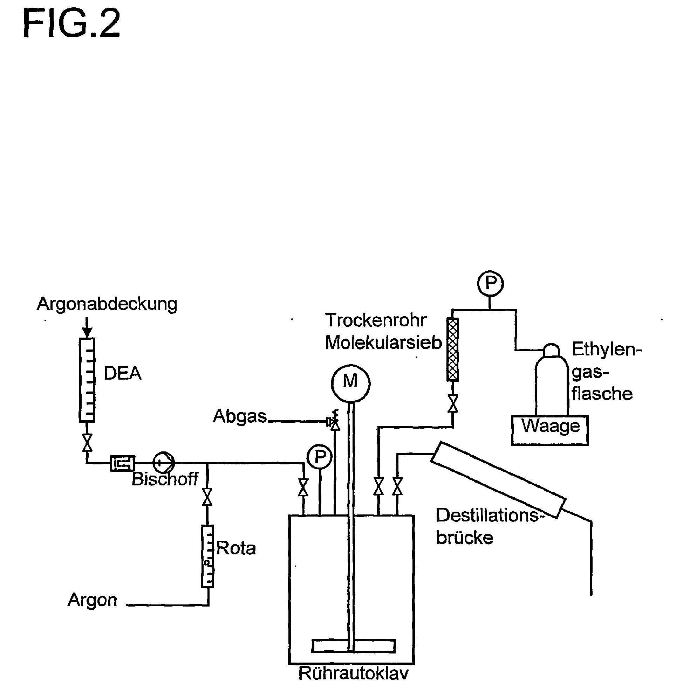

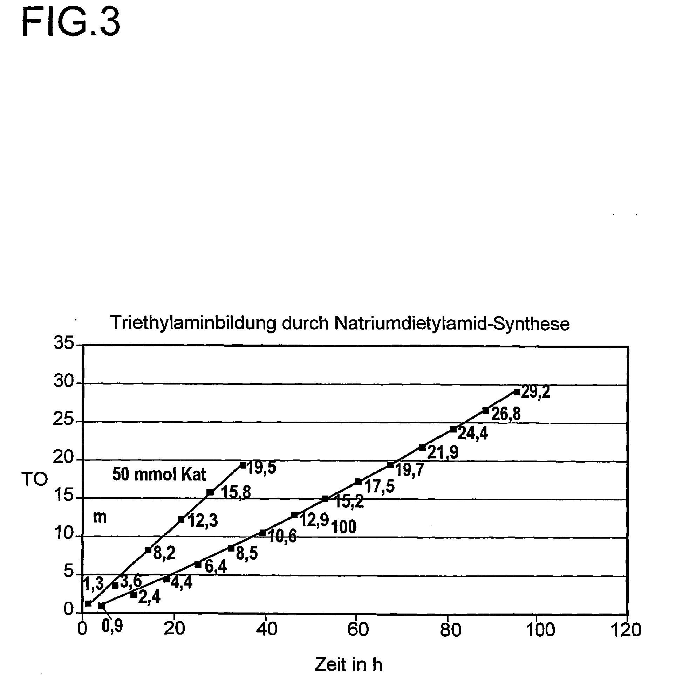

Dabei fiel auf, dass trotz halbierter Katalysatormenge die gleiche Menge an Triethylamin gebildet wurde. Katalytisch aktiv ist, demnach nur das gelöste Natriumdiethylamid. (Siehe Abbildungen 2 rund 3)

### Beispiel 6

### Umsetzung von Diethylamin mit Ethen in Gegenwart von Butyllithium als Katalysator

In den Autoklaven aus Beispiel 2 wurden 40 ml Diethylamin zugefahren und Ethen (5,3 g) aufgepresst. Unter Rühren wurde auf 90 °C erwärmt (Druck: 21 bar) und solange gerührt, bis kein Druckabfall mehr zu beobachten war (7 h) (Druck: 13, 5bar) . NachAbkühlung auf Raumtemperatur wurden drucklos 50 ml Produktgemisch herausdestilliert und GC-analytisch untersucht: Die Lösung enthielt: 71 % DEA und 19 % TEA. Das enspricht 6 g TEA.

### Beispiel 7

### Umsetzung von Triethylamin mit Ammoniak (Isomerisierung)

In einer kontinuierlich betriebenen Laborapparatur (60 ml Rohrreaktor) ohne Rückführung wurde ein Hydrierkatalysator (10Gew.-%CoO, 10 Gew. -% NiO und 4 Gew. -% CuO auf Al₂O₃, gemäß DE-A-19 53 263, Beispiel 1) eingebaut und ohne Aktivierung bei 65 bar Wasserstoffdruck angefahren. Es wurden sowohl Temperatur als auch das Verhältnis von Ammoniak zu Triethylamin imReaktorzulauf variiert. Die Belastung von 0, 2 kg/l*h (kgTriethylamin pro Liter Katalysator und pro Stunde) wurde konstant gelassen:
a) Variation der Temperatur:
   a1)
      In den Reaktor wurden bei 65 bar (23 N1 Wasserstoff (N1 = auf Normalbedingungen umgerechnetes Gasvolumen)) 17 ml/h Ammoniak und 16,55 ml/h Triethylamin (TEA) gefahren. Bei 180 °C erhielt man einen Austrag der ausschließlich aus Monoethylamin (MEA) (25,3 Gew.-%), Diethylamin (DEA) (44,8 Gew.-%) und TEA (29,9 Ge:w.-%) bestand.
   a2)
      In den Reaktor wurden bei 65 bar (23 Nl Wasserstoff) 17 ml/h Ammoniak und 16, 55 ml/h TEA gefahren. Bei 210 °C erhielt man einen Austrag der ausschließlich aus MEA (32,5 gel.-%), DEA (52,3 Gew.-%) und TEA (15,7 Gew.-%) bestand.
b) Variation des NH₃/TEA-Verhältnisses im Reaktorzulauf:
   b1)
      In den Reaktor wurden bei 65 bar (23 N1 Wasserstoff) 3,75 ml/h Ammoniak und 12,4 ml/h TEA gefahren. Bei 200 °C erhielt man einen Austrag der ausschließlich aus MEA (12,6 Gew. -%), DEA (49,9 Gew. -%) und TEA (37,5 Gew.-%) bestand.
   b2) .
      In den Reaktor wurden bei 65 bar (23 N1 Wasserstoff) 15,1 ml/h Ammoniak und 12, 4 ml/h TEA gefahren. Bei 200 °C erhielt man einen Austrag der ausschließlich aus MEA (34, 0 Gew. -%), DEA (49, 7 Gew.-%) und TEA (20, 3 Gew.-%) bestand.
   b3)
      In den Reaktor wurden bei 65 bar (23 N1 Wasserstoff) 30,3 ml/h Ammoniak und 12,4 ml/h TEA gefahren. Bei 200 °C erhielt man einen Austrag der ausschließlich aus MEA (47,2 Gew.-%), DEA (44,7 Gew.-%) und TEA (7,1 Gew.-%) bestand.

### Beispiel 8

### Umsetzung von Triethylamin mit Ammoniak (Isomerisierung) an einem Cu-Kontakt

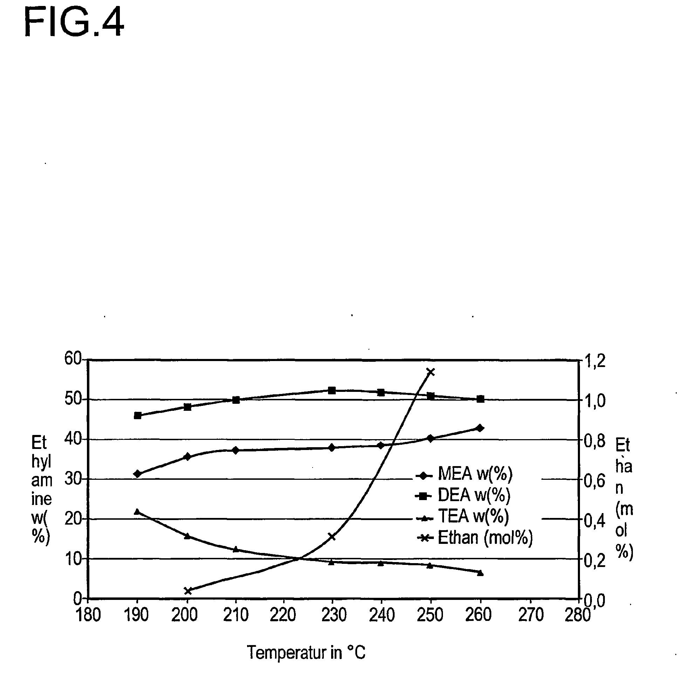

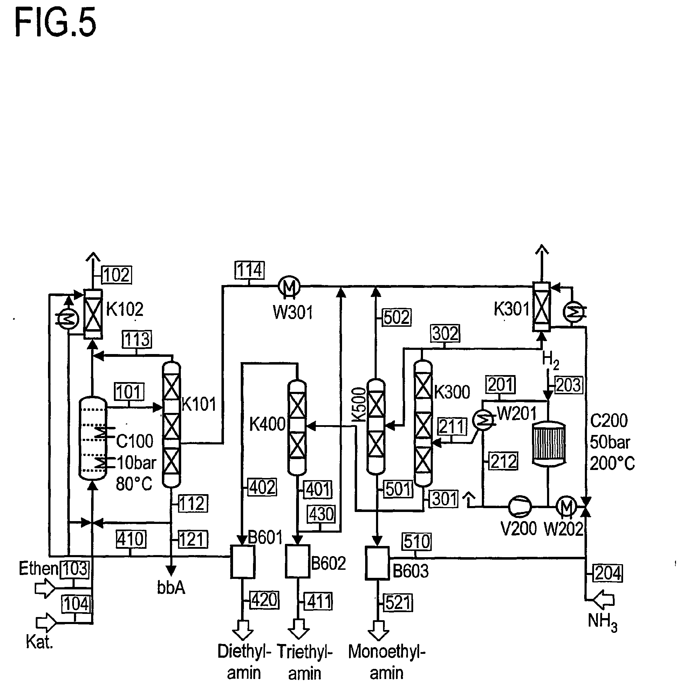

In einer kontinuierlich betriebenen Laborapparatur (Rohrreaktor 60 ml) ohne Rückführung wurde ein Aminierungskatalysator (53 Gew. -⁰₆ CuO auf Al₂O₃) in reduzierter und passivierter Form eingebaut und 6 h bei 200 °C unter Wasserstoffatmosphäre reduziert (Normaldruck) . Dann wurde bei 65 bar Wasserstoffdruck angefahren. Dabei wurden sowohl Temperatur als auch das Verhältnis von Ammoniak zu Triethylamin variiert. Bei einer Belastung von 0,15 kg kg/l*h und einem molaren NH₃/TEA-Verhältnis von 4,3 : 1 wurde die Ethan-Bildung gemessen. Selbst bei hohen Temperaturen von 250 °C blieb die Ethanbildung in einem Bereich von kleiner 1 Mol% (bezogen auf Triethylamin) (siehe folgende Abbildung 4).

| Temp. | Ammoniak | | TEA | | NH3/ TEA | Belast. | H₂ | GC-Analytik | | |
|---|---|---|---|---|---|---|---|---|---|---|
| | | | | | | | | MEA | DEA | TEA |
| in °C | mol/h | g/h | mol/h | ml/h | mol/ mol | kg l*h | Nl/h | | | |
| | | | | | | | | | | |
| 190 | 0,142 | 2,42 | 0,0889 | 12,41 | 1,6 | 0,15 | 23 | 19,0 | 48,5 | 32,5 |
| 200 | 0,142 | 2,42 | 0,0889 | 12,41 | 1,6 | 0,15 | 23 | 19,8 | 51,1 | 29,1 |
| 210 | 0,142 | 2,42 | 0,0889 | 12,41 | 1,6 | 0,15 | 23 | 22,6 | 53,2 | 24,2 |
| 230 | 0,142 | 2,42 | 0,0889 | 12,41 | 1,6 | 0,15 | 23 | 23,3 | 56,7 | 20,0 |
| | | | | | | | | | | |
| 190 | 0,234 | 4 | 0,0889 | 12,41 | 2,6 | 0,15 | 23 | 23,6 | 47,1 | 29,3 |
| 200 | 0,234 | 4 | 0,0889 | 12,41 | 2,6 | 0,15 | 23 | 27,3 | 50,5 | 21,9 |
| 210 | 0,234 | 4 | 0,0889 | 12,41 | 2,6 | 0,15 | 23 | 28,7 | 52,6 | 18,7 |
| 230 | 0,234 | 4 | 0,0889 | 12,41 | 2,6 | 0,15 | 23 | 32,9 | 54,6 | 12,3 |
| | | | | | | | | | | |
| 190 | 0,38 | 6,5 | 0,0889 | 12,41 | 4,3 | 0,15 | 23 | 31,3 | 46 | 21,9 |
| 200 | 0,38 | 6,5 | 0,0889 | 12,41 | 4,3 | 0,15 | 23 | 35,6 | 48,2 | 15,7 |
| 210 | 0,38 | 6,5 | 0,0889 | 12,41 | 4,3 | 0,15 | 23 | 37,3 | 49,9 | 12,3 |
| 230 | 0,38 | 6,5 | 0,0889 | 12,41 | 4,3 | 0,15 | 23 | 38,1 | 52,3 | 9,2 |
| 240 | 0,38 | 6,5 | 0,0889 | 12,41 | 4,3 | 0,15 | 23 | 38,6 | 51,9 | 9,1 |
| 250 | 0,38 | 6,5 | 0,0889 | 12,41 | 4,3 | 0,15 | 23 | 40,3 | 51 | 8,4 |
| 260 | 0,38 | 6,5 | 0,0889 | 12,41 | 4,3 | 0,15 | 23 | 42,9 | 50,1 | 6,5 |

### Erläuterungen zu obiger Ergebnistabelle:

Die Belastung (Belast.) des Katalysators wurde dabei konstant bei 0,15 kg Triethylamin pro Liter Katalysator und pro Stunde (kg/l*h) belassen.

Die Wasserstoffmenge betrug 23 Normliter pro Stunde (Normliter = Nl = auf Normalbedingungen umgerechnetes Gasvolumen) GC-Analytik: Angaben für die Komponenten MEA (Monoethylamin), DEA (Diethylamin) und TEA (Triethylamin) in GC-Flächen-% (GC-Säule: Kapillarsäule30mlang, 1,5ocm, 0,32mmRtx-5-Amine, Temperaturprogramm: 50 °C (5 Min.), dann Aufheizung mit 15 °C/Min.).

### Beispiel 9

### Isomerisierung von Dibutylbutenylamin-Gemischen aus der Reaktion von Dibutylamin und Butadien

In einem kontinuierlich betriebenen Rohreaktor (Länge 30 cm, Volumen 130 cm³, Kat.-Volumen: 50 ml) wurde bei 10 bar Wasserstoffdruck (10 Nl) und einer Belastung von 0,3 kg/l*h des Reaktionsaustrages einer Umsetzung von Butadien mit Di-n-butylamin (Beispiel 3) der Anteil des Ammoniaks zwischen 0,65 bis 2,69 Mol-Verhältnissen variiert. Bei einer Temperatur von 230 °C wurde der in Beispiel 8 beschriebene Cu-Katalysator verwendet. Der Reaktionsaustrag wurde gaschromatographisch (wie in Beispiel 8) analysiert. Die Verhältnisse der drei Amine Monobutylamin (MBA), Dibutylamin (DBA) und Tributylamin (TBA) stiegen zu Gunsten des MBAs bei zunehmender Ammoniakmenge (siehe Tabelle).

| Temp. in °C | Druck in bar | H2 Nl/h | Edukt g/h | Edukt mmol/ h | NH3 g/h | NH3 mmol/h | NH3/ DBBA | MBA in % | DBA in % | TBA in % |
|---|---|---|---|---|---|---|---|---|---|---|
| 230 | 10 | 10 | 15 | 81 | - | - | - | 0,56 | 18,34 | 66,05 |
| 230 | 10 | 10 | 15 | 81 | 0,9 | 53 | 0,65 | 8,19 | 44,17 | 33,54 |
| 230 | 10 | 10 | 15 | 81 | 2,6 | 153 | 1,89 | 17,97 | 48,93 | 19,31 |
| 230 | 10 | 10 | 15 | 81 | 3,7 | 218 | 2,69 | 22,03 | 48,71 | 15 |

| | | | | | | | | | | |
|---|---|---|---|---|---|---|---|---|---|---|
| (DBBA = Dibutylbutylenamin) | | | | | | | | | | |

### Beispiel 10

### Herstellung von Dibutylbutenylamin ("Eintopf") und anschließende Isomerisierung

50 mmol Na wurden in 30 ml wasserfreiem n-Octan bei 110 °C gesclimolzen und durch Rühren dispergiert. Nach dem Abkühlen wurden 476,4 g über 3Å-Molsieb getrocknetes Di-n-Butylaminzugegeben und unter Rühren bei Normaldruck 4 1/h Butadien übergeleitet. Nach 1,5 h wurde die Temperatur auf 60 °C erhöht und weitere 8 h Butadien übergeleitet. Dann wurde die Reaktion durch Zugabe von 30 ml wässriger 30 Gew. -%iger NaOH gestoppt, die organische Phase abgetrennt, mit 3Å-Molsieb getrocknet und via GC analysiert.

Ergebnis:

| | |
|---|---|
| Substanz | GC-Fl.% |
| Butadien | 0,155 |
| 2-Butene | 0,064 |
| Di-n-butylamin | 21,818 |
| 4-Vinylcyclohexen- | 0,118 |
| Di-n-Butylbutenylamine | 77,823 |

Das vorliegende Gemisch wurde wie in Versuch 9 mit Ammoniak (NH₃ zu Di-n-butylbut-2-(E/Z)enyl / Di-n-butylbut-1-(E/Z)enyl / Di-n-butyl-but-3-enyl - Gemisch = 1,67 zu 1) mit einer Belastung von 0,3 kg/l*h und 230 °C/10 bar/10 Nl H₂ über den inBeispiel 8 beschriebenen Cu-Kontakt gefahren. Dabei erhielt man ein Reaktionsgemisch enthaltend 14,3 Gew.-% Mono-n-butylamin (MBA), 53,9 Gew.-% Di-n-butylamin (DBA) und 30,4 Gew.-% Tri-n-butylamin (TBA).

### Vorbemerkungen zu den Beispielen 11 bis 14

Basis der im folgenden aufgeführten Beispiele sind für die 1. und 2. Verfahrensstufe diskontinuierliche Autoklavenversuche (Beispiele 4, 5 und 6 bzgl. der 1. Verfahrensstufe) und kontinuierliche Umalkylierungsversuche (Beispiele 7 und 8 bzgl. der 2. Verfahrensstufe).

Die Aufarbeitung der Reaktionsausträge wurde mit Hilfe thermodynamischer Daten simuliert. Die thermodynamischen Daten der Reinstoffe stammen aus der DIPPR-Datenbank [Design Institute of Physical Properties Data, Version 11.0]. Die binären Systeme wurden mit einemNRTL-AspenAnsatz beschrieben. DemAnsatz wurden sowohl eigene Messreihen als auch UNIFAC-Abschätzungen (Inkrementenmethode) zugrunde gelegt. Die Energie und Stoffbilanzen wurden mit einem Gleichungslöser berechnet. Die angegebenen Stromnummern beziehen sich auf das Blockschaltbild Abb. 1. Das der Simulation zugrunde gelegte Verfahrensschema wird im Anhang 1 gezeigt. Das Verfahrensschema gilt für die Beispiele 11-14. Die Strommengen und Stromzusammensetzungen gelten jedoch nur für Beispiel 11.

### Beispiel 11

### Herstellung von 80 kg/h Monoethylamin, 27,5 kg/h Diethylamin und 80 kg/h Triethylamin

| Strom | | 1 | 2 | 3 | 4 | 5 | 6 | 7 | 8 | 9 | 10 | 11 |
|---|---|---|---|---|---|---|---|---|---|---|---|---|
| Menge | [kg/h] | 137,5 | 58,6 | 515,6 | 378,3 | 875,1 | 825 | 50,2 | 66,0 | 80,0 | 27,5 | 80,0 |
| Monoethylamin | [kg/h] | 0,0 | 0,0 | 0,1 | 0,1 | 125,7 | 45,8 | 0,0 | 10,0 | 0,0 | 0,0 | 79,9 |
| Diethylamin | [kg/h] | 0,0 | 20,4 | 19,4 | 377,8 | 405,6 | 19,7 | 0,0 | 8,1 | 0,1 | 27,5 | 0,0 |
| Triethylamin | [kg/h] | 0,0 | 24,4 | 496,1 | 0,4 | 310,1 | 725,9 | 0,0 | 2,0 | 79,9 | 0,0 | 0,0 |
| Ammoniak | [kg/h] | 0,0 | 0,0 | 0,0 | 0,0 | 33,6 | 33,6 | 50,2 | 16,4 | 0,0 | 0,0 | 0,1 |
| Ethen | [kg/h] | 137,5 | 8,0 | 0,0 | 0,0 | 0,0 | 0,0 | 0,0 | 0,0 | 0,0 | 0,0 | 0,0 |
| Kat. (1.Stufe) | [kg/h] | 0,0 | 5,7 | 0,0 | 0,0 | 0,0 | 0,0 | 0,0 | 0,0 | 0,0 | 0,0 | 0,0 |
| H2 | [kg/h] | 0,0 | 0,0 | 0,0 | 0,0 | 0,0 | 0,0 | 0,0 | 29,6 | 0,0 | 0,0 | 0,0 |

Der Reaktoreintrittsstrom der 2. Verfahrensstufe setzt sich bzgl. der Hauptkomponenten wie folgt zusammen: Monoethylamin: 5,9 Gew.-%, Diethylamin: 2,9 Gew.-%, Triethylamin: 77,3 Gew.-%, Ammoniak: 10,6 Gew.-%, Wasserstoff 3,1 Gew.-%.

### Beispiel 12

### Herstellung von 187,5 kg/h Monoethylamin

| Strom | | 1 | 2 | 3 | 4 | 5 | 6 | 7 | 8 | 9 | 10 | 11 |
|---|---|---|---|---|---|---|---|---|---|---|---|---|
| Menge | [kg/h] | 116,7 | 49,7 | 437,6 | 321,0 | 882,3 | 811 | 71,0 | 126,4 | 0,0 | 0,0 | 187,5 |
| Monoethylamin | [kg/h] | 0,0 | 0,0 | 0,0 | 0,1 | 212,1 | 24,8 | 0,0 | 14,6 | 0,0 | 0,0 | 187,3 |
| Diethylamin | [kg/h] | 0,0 | 17,3 | 16,5 | 320,6 | 320,9 | 16,7 | 0,0 | 5,5 | 0,0 | 0,0 | 0,0 |
| Triethylamin | [kg/h] | 0,0 | 20,7 | 421,0 | 0,3 | 180,2 | 600,9 | 0,0 | 1,0 | 0,0 | 0,0 | 0,0 |
| Ammoniak | [kg/h] | 0,0 | 0,0 | 0,0 | 0,0 | 169,1 | 168,9 | 71,0 | 71,2 | 0,0 | 0,0 | 0,2 |
| Ethen | [kg/h] | 116,7 | 6,8 | 0,0 | 0,0 | 0,0 | 0,0 | 0,0 | 0,0 | 0,0 | 0,0 | 0,0 |
| Kat.(1.Stufe) | [kg/h] | 0,0 | 4,9 | 0,0 | 0,0 | 0,0 | 0,0 | 0,0 | 0,0 | 0,0 | 0,0 | 0,0 |
| H2 | [kg/h] | 0,0 | 0,0 | 0,0 | 0,0 | 0,0 | 0,0 | 0,0 | 34,1 | 0,0 | 0,0 | 0,0 |

Der Reaktoreintrittsstrom der 2. Verfahrensstufe setzt sich bzgl. der Hauptkomponenten wie folgt zusammen: Monoethylamin: 3,9 Gew.-%, Diethylamin: 2,2 Gew.-%, Triethylamin: 59,6 Gew.-%, Ammoniak: 30,8 Ges.-%, Wasserstoff 3,4 Ges.-%.

### Beispiel 13

### Herstellung von 0,1875 t/h Diethylamin

| Strom | | 1 | 2 | 3 | 4 | 5 | 6 | 7 | 8 | 9 | 10 | 11 |
|---|---|---|---|---|---|---|---|---|---|---|---|---|
| Menge | [kg/h] | 144,0 | 61,3 | 540,0 | 396,1 | 1258,7 | 1215 | 43,7 | 94,9 | 0,0 | 187,5 | 0,0 |
| Monoethylamin | [kg/h] | 0,0 | 0,0 | 0,1 | 0,1 | 180,8 | 180,8 | 0,0 | 14,4 | 0,0 | 0,0 | 0,0 |
| Diethylamin | [kg/h] | 0,0 | 21,3 | 20,4 | 395,7 | 583,4 | 20,8 | 0,0 | 11,6 | 0,0 | 187,3 | 0,0 |
| Triethylamin | [kg/h] | 0,0 | 25,6 | 519,5 | 0,4 | 446,1 | 965,0 | 0,0 | 2,8 | 0,0 | 0,2 | 0,0 |
| Ammoniak | [kg/h] | 0,0 | 0,0 | 0,0 | 0,0 | 48,4 | 48,4 | 43,7 | 23,6 | 0,0 | 0,0 | 0,0 |
| Ethen | [kg/h] | 144,0 | 8,4 | 0,0 | 0,0 | 0,0 | 0,0 | 0,0 | 0,0 | 0,0 | 0,0 | 0,0 |
| Kat. (1.Stufe) | [kg/h] | 0,0 | 6,0 | 0,0 | 0,0 | 0,0 | 0,0 | 0,0 | 0,0 | 0,0 | 0,0 | 0,0 |
| H2 | [kg/h] | 0,0 | 0,0 | 0,0 | 0,0 | 0,0 | 0,0 | 0,0 | 42,5 | 0,0 | 0,0 | 0,0 |

Der Reaktoreintrittsstrom der 2. Verfahrensstufe setzt sich bzgl. der Hauptkomponenten wie folgt zusammen: Monoethylamin: 14,4 Gew.-%, Diethylamin: 2,4 Gew.-%, Triethylamin: 71,5 Gew.-%, Ammoniak: 8,5 Ges.-%, Wasserstoff 3,1 Ges.-%.

### Beispiel 14

### Herstellung von 0,1875 t/h Triethylamin

| Strom | | 1 | 2 | 3 | 4 | 5 | 6 | 7 | 8 | 9 | 10 | 11 |
|---|---|---|---|---|---|---|---|---|---|---|---|---|
| Menge | [kg/h] | 156,1 | 66,5 | 585,3 | 429,4 | 882,9 | 851 | 31,6 | 49,2 | 187,5 | 0,0 | 0,0 |
| META | [kg/h] | 0,0 | 0,1 | 0,1 | 0,1 | 88,8 | 88,8 | 0,0 | 6,5 | 0,0 | 0,0 | 0,0 |
| DEA | [kg/h] | 0,0 | 23,1 | 22,1 | 428,9 | 429,3 | 22,2 | 0,0 | 7,9 | 0,2 | 0,0 | 0,0 |
| TEA | [kg/h] | 0,0 | 27,7 | 563,2 | 0,4 | 349,8 | 725,3 | 0,0 | 2,1 | 187,3 | 0,0 | 0,0 |
| NH3 | [kg/h] | 0,0 | 0,0 | 0,0 | 0,0 | 15,1 | 15,1 | 31,6 | 6,8 | 0,0 | 0,0 | 0,0 |
| ETHEN | [kg/h] | 156,1 | 9,1 | 0,0 | 0,0 | 0,0 | 0,0 | 0,0 | 0,0 | 0,0 | 0,0 | 0,0 |
| KAT | [kg/h] | 0,0 | 6,5 | 0,0 | 0,0 | 0,0 | 0,0 | 0,0 | 0,0 | 0,0 | 0,0 | 0,0 |
| H2 | [kg/h] | 0,0 | 0,0 | 0,0 | 0,0 | 0,0 | 0,0 | 0,0 | 25,9 | 0,0 | 0,0 | 0,0 |

Der Reaktoreintrittsstrom der 2. Verfahrensstufe setzt sich bzgl. der Hauptkomponenten wie folgt zusammen: Monoethylamin: 10,2 Gew.-%, Diethylamin: 3,2 Gew.-%, Triethylamin: 78,1 Gew.-%, Ammoniak: 5,6 Gew.-%, Wasserstoff 2,8 Gew.-%.

### Zur Abbildung im Anhang 1:

Es handelt sich um eine simulierte Verfahrensvariante unter Berücksichtigung der wesentlichen Stoffe. Die Darstellung ist vereinfacht. Die Zusammensetzung der Ströme bezieht sich auf Beispiel 11. Die Stromnummern (1 - 11) gemäß dem Blockschaltbild Abb. 1 entsprechen wie folgt den obigen Stromnummern:
1 = 103, 2 = 112 +113, 3 =114, 4 = 410, 5 = 211, 6 = 502 + 430 + 114, 7 = 204, 8 = 212, 9 = 411, 10 = 420, 11 = 521.

Die 1. Verfahrensstufe besteht aus dem Reaktor C100, der Kolonne K101 (Kopfdruck: 5 bar, Kopftemperatur: 88°C, Sumpftemperatur: 154°C, theoretische Stufen: 10) und dem Absorber K102. Die zweite Verfahrensstufe besteht aus dem Reaktor C200, dem Kondensator W201, dem Verdampfer W202, dem Verdichter V200 und dem Absorber K301. Die Aufarbeitung besteht aus der Sequenz der Destillationskolonnen K300 (Kopfdruck: 5 bar, Kopftemperatur: 50°C, Sumpftemperatur: 123°C, theoretische Stufenzahl: 18), K400 (Kopfdruck: 2 bar, Kopftemperatur: 77°C, Sumpftemperatur: 114°C, theoretische Stufenzahl: 15) und K500 (Kopfdruck: 5 bar, Kopftemperatur: 36°C, Sumpf temperatur: 64°C, theoretische Stufenzahl: 6).

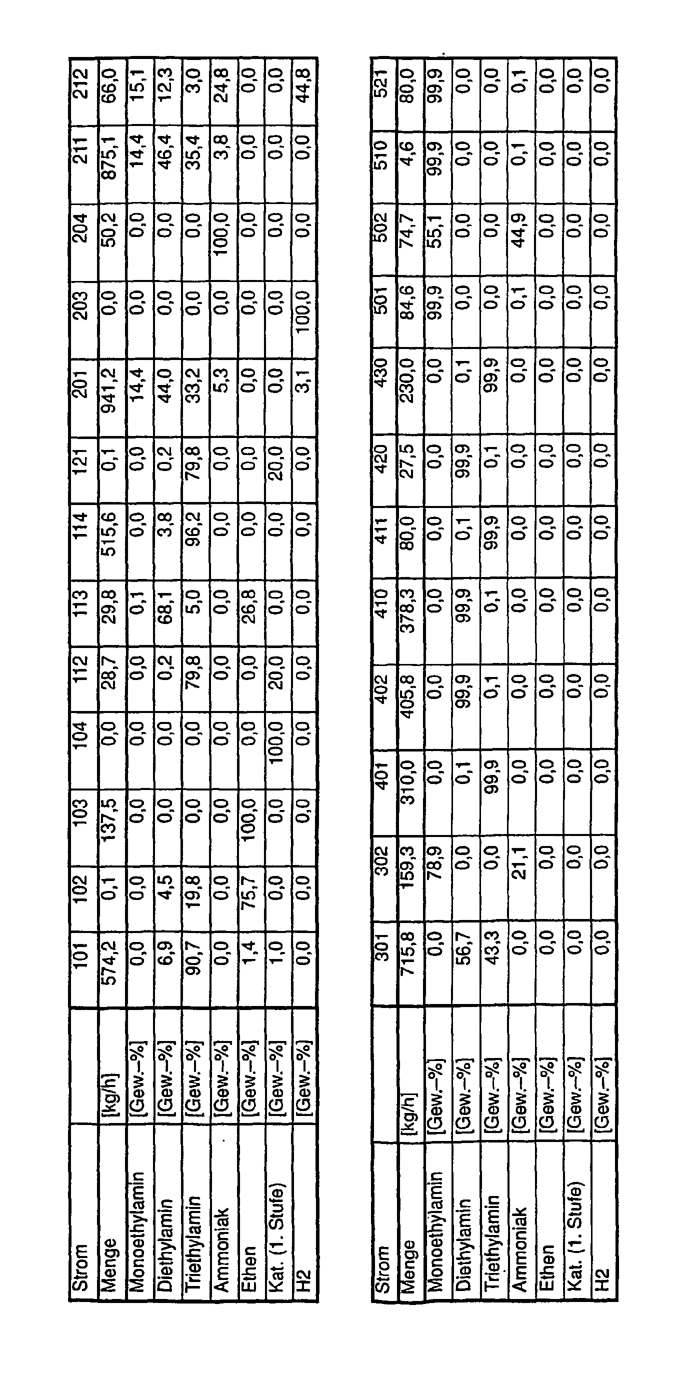

## Patentansprüche

1. Verfahren zur Herstellung von Alkylaminen, **dadurch gekennzeichnet, dass** man in einer ersten Verfahrensstufe ein Olefin
a) mit einem primären Amin und/oder einem sekundären Amin in Gegenwart eines Metallmonoalkylamids oder Metalldialkylamids als Katalysator oder
b) mit Ammoniak und/oder einem primären Amin in Gegenwart einer anorganischen Festkörpersäure als Katalysator oder
c) mit Ammoniak, einem primären Amin und/oder einem sekundären Amin in Gegenwart einer Übergangsmetallkomplexverbindung als Katalysator,
unter hydroaminierenden Bedingungen umsetzt und anschließend das oder die erhaltene/n Hydroaminierungsprodukt/e in der zweiten Verfahrensstufe unter umalkylierenden Bedingungen
d) in Gegenwart eines Umalkylierungskatalysators oder
e) in Gegenwart von Wasserstoff und eines umalkylierenden Hydrier- oder Dehydrierkatalysators
bei Temperaturen von 80 bis 400 °C umsetzt.

2. Verfahren nach Anspruch 1, **dadurch gekennzeichnet, dass** in der Bilanz von den Einsatzstoffen Olefin und Ammoniak, primäres Amin und/oder sekundäres Amin nur Ammoniak und Olefin verbraucht werden.

3. Verfahren nach den Ansprüchen 1 oder 2, **dadurch gekennzeichnet, dass** man die Umsetzung in der zweiten Verfahrensstufe unter umalkylierenden und hydrierenden Bedingungen durchführt.

4. Verfahren nach einem der Ansprüche 1 bis 3, **dadurch gekennzeichnet, dass** man die Umsetzung in der zweiten Verfahrensstufe in Gegenwart von Ammoniak durchführt.

5. Verfahren nach einem der Ansprüche 1 bis 4, **dadurch gekennzeichnet, dass** man das in der zweiten Verfahrensstufe erhaltene Produktgemisch auftrennt in Produkt/e, das/die in die erste Verfahrensstufe zurückgeführt werden, und/oder Produkt/e, das/die in die zweite Verfahrensstufe zurückgeführt werden, und das oder die gewünschten Alkylamin/e.

6. Verfahren nach einem der Ansprüche 1 bis 5, **dadurch gekennzeichnet, dass** man die Umsetzung in der zweiten Verfahrensstufe in Gegenwart von Wasserstoff und eines umalkylierenden Hydrier- oder Dehydrierkatalysators durchführt, wobei im gleichen Verfahrensschrit ungesättigte Alkylamine und/oder entsprechende Imine zu den entsprechenden Alkylaminen hydriert werden.

7. Verfahren nach einem der Ansprüche 1 bis 6, **dadurch gekennzeichnet**,5dass man die Umsetzung in der zweiten Verfahrensstufe kontinuierlich in Gegenwart eines Hydrier- oder Dehydrierkatalysators im Festbett durchführt.

8. Verfahren nach einem der Ansprüche 1 bis 7, **dadurch gekennzeichnet, dass** man die Umsetzung in der zweiten Verfahrensstufe in Gegenwart eines kupferhaltigen Katalysators, der ein Trägermaterial enthalten kann, und Wasserstoff durchführt.

9. Verfahren nach einem der Ansprüche 1 bis 8, **dadurch gekennzeichnet, dass** man die Umsetzung in der ersten Verfahrensstufe in Gegenwart eines Alkalimetalldialkylamids oder Zeoliths als Katalysator durchführt.

10. Verfahren nach einem der Ansprüche 1 bis 9, **dadurch gekennzeichnet, dass** man die Umsetzung in der ersten Verfahrensstufe in Gegenwart eines Natriumdialkylamids als Katalysator durchführt.

11. Verfahren nach einem der Ansprüche 1 bis 10, **dadurch gekennzeichnet, dass** man in der ersten Verfahrensstufe ein Olefin mit einem primären Amin und/oder einem sekundären Amin in Gegenwart eines Metallmonoalkylamids oder Metalldialkylamids oder einer Übergangsmetallkomplexverbindung als Katalysator umsetzt und anschließend das oder die erhaltene/n Hydroaminierungsprodukt/e in der zweiten Verfahrensstufe in Gegenwart von Ammoniak und unter Umsatz des Ammoniaks unter umalkylierenden Bedingungen umsetzt und anschließend das in der zweiten Verfahrensstufe erhaltene Produktgemisch auftrennt in Produkt/e, das/die in die erste Verfahrensstufe zurückgeführt werden, und/oder Produkt/e, das/die in die zweite Verfahrensstufe zurückgeführt werden, und das oder die gewünschten Alkylamin/e.

12. Verfahren nach einem der Ansprüche 1 bis 10, **dadurch gekennzeichnet, dass** man in der ersten Verfahrensstufe ein Olefin mit Ammoniak, einem primären Amin und/oder einem sekundären Amin unter hydroaminierenden Bedingungen umsetzt und anschließend das oder die erhaltene/n Hydroaminierungsprodukt/e in der zweiten Verfahrensstufe in Gegenwart von Ammoniak und/oder dem primären Amin unter umalkylierenden Bedingungen umsetzt.

13. Verfahren nach einem der Ansprüche 1 bis 12, **dadurch gekennzeichnet, dass** es stich bei dem Olefin um Ethen, bei dem primären Amin um Monoethylamin, bei dem sekundären Amin um Diethylamin und bei den verfahrensgemäß hergestellten Alkylaminen um Monoethylamin, Diethylamin und Triethylamin handelt.

14. Verfahren nach einem der Ansprüche 1 bis 12, **dadurch gekennzeichnet, dass** es sich bei dem Olefin um 1,3-Butadien, bei dem primären Amin um Mono-n-butylamin, bei dem sekundären Amin um Di-n-butylamin und bei den verfahrensgemäß hergestellten Alkylaminen um Mono-n-butylamin, Di-n-butylamin und Tri-n-butylamin handelt.

15. Verfahren nach einem der Ansprüche 1 bis 12, **dadurch gekennzeichnet, dass** es sich bei dem Olefin um Propen, bei dem primären Amin um Monoisopropylamin und bei den verfahrensgemäß hergestellten Alkylaminen um Monoisopropylamin und Diisopropylamin handelt.

## Claims

1. A process for the preparation of alkylamines, which comprises, in a first process stage, reacting an olefin
a) with a primary amine and/or a secondary amine in the presence of a metal monoalkylamide or metal dialkylamide as catalyst or
b) with ammonia and/or a primary amine in the presence of an inorganic solid-state acid as catalyst or
c) with ammonia, a primary amine and/or a secondary amine in the presence of a transition metal complex compound as catalyst,
under hydroaminating conditions, and then, in a second process stage, reacting the resulting hydroamination product(s) under transalkylating conditions
d) in the presence of a transalkylating catalyst or
e) in the presence of hydrogen and a transalkylating hydrogenation or dehydrogenation catalyst
at temperatures of from 80 to 400°C.

2. The process according to claim 1, wherein, in the balance of the feed materials olefin and ammonia, primary amine and/or secondary amine, only ammonia and olefin are consumed.

3. The process according to claim 1 or 2, wherein the reaction in the second process stage is carried out under transalkylating and hydrogenating conditions.

4. The process according to any of claims 1 to 3, wherein the reaction in the second process stage is carried out in the presence of ammonia.

5. The process according to any of claims 1 to 4, wherein the product mixture obtained in the second process stage is separated into product(s) which is/are returned to the first process stage, and/or product(s) which is/are returned to the second process stage, and the desired alkylamine(s).

6. The process according to any of claims 1 to 5, wherein the reaction in the second process stage is carried out in the presence of hydrogen and a transalkylating hydrogenation or dehydrogenation catalyst, where, in the same process step, unsaturated alkylamines and/or corresponding imines are hydrogenated to give the corresponding alkylamines.

7. The process according to any of claims 1 to 6, wherein the reaction in the second process stage is carried out continuously in the presence of a hydrogenation or dehydrogenation catalyst in a fixed bed.

8. The process according to any of claims 1 to 7, wherein the reaction in the second process stage is carried out in the presence of a copper-containing catalyst, which may comprise a support material, and hydrogen.

9. The process according to any of claims 1 to 8, wherein the reaction in the first process stage is carried out in the presence of an alkali metal dialkylamide or zeolite as catalyst.

10. The process according to any of claims 1 to 9, wherein the reaction in the first process stage is carried out in the presence of a sodium dialkylamide as catalyst.

11. The process according to any of claims 1 to 10, wherein, in the first process stage, an olefin is reacted with a primary amine and/or a secondary amine in the presence of a metal monoalkylamide or metal dialkylamide or a transition metal complex compound as catalyst, and then, in the second process stage, the resulting hydroamination product(s) is/are reacted in the presence of ammonia and with conversion of the ammonia under transalkylating conditions, and then the product mixture obtained in the second process stage is separated into product(s) which is/are returned to the first process stage, and/or product(s) which is/are returned to the second process stage, and the desired alkylamine(s).

12. The process according to any of claims 1 to 10, wherein, in the first process stage, an olefin is reacted with ammonia, a primary amine and/or a secondary amine under hydroaminating conditions, and then, in the second process stage, the resulting hydroamination product(s) is/are reacted in the presence of ammonia and/or the primary amine under transalkylating conditions.

13. The process according to any of claims 1 to 12, wherein the olefin is ethene, the primary amine is monoethylamine, the secondary amine is diethylamine and the alkylamines prepared in accordance with the process are monoethylamine, diethylamine and triethylamine.

14. The process according to any of claims 1 to 12, wherein the olefin is 1,3-butadiene, the primary amine is mono-n-butylamine, the secondary amine is di-n-butylamine and the alkylamines prepared in accordance with the process are mono-n-butylamine, di-n-butylamine and tri-n-butylamine.

15. The process according to any of claims 1 to 12, wherein the olefin is propene, the primary amine is monoisopropylamine and the alkylamines prepared in accordance with the process are monoisopropylamine and diisopropylamine.

## Revendications

1. Procédé pour la préparation d'alkylamines, **caractérisé en ce qu'**on transforme, dans une première étape de procédé, une oléfine
a) avec une amine primaire et/ou une amine secondaire en présence d'un monoalkylamide de métal ou d'un dialkylamide de métal comme catalyseur ou
b) avec de l'ammoniaque et/ou une amine primaire en présence d'un acide inorganique solide comme catalyseur ou
c) avec de l'ammoniaque, une amine primaire et/ou une amine secondaire en présence d'un composé complexe
de métal de transition comme catalyseur, dans des conditions d'hydroamination puis on transforme le/les produit(s) d'hydroamination obtenu(s) dans une deuxième étape de procédé dans des conditions de transalkylation
d) en présence d'un catalyseur de transalkylation ou
e) en présence d'hydrogène et d'un catalyseur de transalkylation d'hydrogénation ou de déshydrogénation
à des températures de 80 à 400°C.

2. Procédé selon la revendication 1, **caractérisé en ce que** dans le bilan des substances de départ oléfine et ammoniac, amine primaire et/ou amine secondaire, on ne consomme que l'ammoniaque et l'oléfine.

3. Procédé selon les revendications 1 ou 2,
**caractérisé en ce qu'**on réalise la transformation dans la deuxième étape de procédé dans des conditions de transalkylation et d'hydrogénation.

4. Procédé selon l'une quelconque des revendications 1 à 3, **caractérisé en ce qu'**on réalise la transformation dans la deuxième étape de procédé en présence d'ammoniaque.

5. Procédé selon l'une quelconque des revendications 1 à 4, **caractérisé en ce qu'**on sépare le mélange de produits obtenu dans la deuxième étape de procédé en produit(s), qui est/sont recyclé(s) dans la première étape de procédé et/ou en produit(s) qui est/sont recyclé(s) dans la deuxième étape de procédé et en la/les alkylamine(s) souhaitée(s).

6. Procédé selon l'une quelconque des revendications 1 à 5, **caractérisé en ce qu'**on réalise la transformation dans la deuxième étape de procédé en présence d'hydrogène et d'un catalyseur de transalkylation d'hydrogénation ou de déshydrogénation, où on hydrogène dans la même étape de procédé des alkylamines insaturées et/ou des imines correspondantes en alkylamines correspondantes.

7. Procédé selon l'une quelconque des revendications 1 à 6, **caractérisé en ce qu'**on réalise la transformation dans la deuxième étape de procédé en continu en présence d'un catalyseur d'hydrogénation ou de déshydrogénation dans un lit fixe.

8. Procédé selon l'une quelconque des revendications 1 à 7, **caractérisé en ce qu'**on réalise la transformation dans la deuxième étape de procédé en présence d'un catalyseur contenant du cuivre, qui peut contenir un matériau support, et d'hydrogène.

9. Procédé selon l'une quelconque des revendications 1 à 8, **caractérisé en ce qu'**on réalise la transformation dans la première étape de procédé en présence d'un dialkylamide de métal alcalin ou d'une zéolithe comme catalyseur.

10. Procédé selon l'une quelconque des revendications 1 à 9, **caractérisé en ce qu'**on réalise la transformation dans la première étape de procédé en présence d'un dialkylamide de sodium comme catalyseur.

11. Procédé selon l'une quelconque des revendications 1 à 10, **caractérisé en ce qu'**on transforme, dans la première étape de procédé, une oléfine avec une amine primaire et/ou une amine secondaire en présence d'un monoalkylamide de métal ou d'un dialkylamide de métal ou d'un composé complexe de métal de transition comme catalyseur puis on transforme le ou les produit(s) d'hydroamination obtenu(s) dans la deuxième étape de procédé en présence d'ammoniaque et avec transformation de l'ammoniaque dans des conditions de transalkylation, puis on sépare le mélange de produits obtenu dans la deuxième étape de procédé en produit(s) qui est/sont recyclê(s) dans la première étape de procédé et/ou en produit(s) qui est/sont recyclé(s) dans la deuxième étape de procédé et en la ou les alkylamine(s) souhaitée(s).

12. Procédé selon l'une quelconque des revendications 1 à 10, **caractérisé en ce qu'**on transforme dans la première étape de procédé une oléfine avec de l'ammoniaque, une amine primaire et/ou une amine secondaire dans des conditions d'hydroamination, puis on transforme le/les produit(s) d'hydroamination obtenu(s) dans la deuxième étape de procédé en présence d'ammoniaque et/ou de l'amine primaire dans des conditions de transalkylation.

13. Procédé selon l'une quelconque des revendications 1 à 12, **caractérisé en ce qu'**il s'agit, pour l'oléfine, d'éthylène, pour l'amine primaire, de monoéthylamine, pour l'amine secondaire, de diéthylamine et pour les alkylamines préparées selon le procédé de monoéthylamine, de diéthylamine et de triéthylamine.

14. Procédé selon l'une quelconque des revendications 1 à 12, **caractérisé en ce qu'**il s'agit, pour l'oléfine, de 1,3-butadiène, pour l'amine primaire, de mono-n-butylamine, pour l'amine secondaire, de di-n-butylamine et pour les alkylamines préparées selon le procédé de mono-n-butylamine, de di-n-butylamine et de tri-n-butylamine.

15. Procédé selon l'une quelconque des revendications 1 à 12, **caractérisé en ce qu'**il s'agit, pour l'oléfine, de propylène, pour l'amine primaire, de mono-isopropylamine et pour les alkylamines préparées selon le procédé de mono-isopropylamine et de triisopropylamine.
